# EUROPEAN PATENT SPECIFICATION

(11) **EP 1 360 495 B1**
(45) Date of publication and mention of the grant of the patent: **11.04.2007**
(21) Application number: 01944494.2
(22) Date of filing: 13.06.2001
(51) Int. Cl.: G01N 33/548

(54) **HIGH THROUGHPUT SCREENING OF COMPOUNDS FOR BIOLOGICAL ACTIVITY**
HOCHDURCHSATZSCREENING VON VERBINDUNGEN FÜR BIOLOGISCHE WIRKSAMKEIT
PROCEDE DE CRIBLAGE A HAUT RENDEMENT DE COMPOSES POTENTIELS POUR UNE ACTIVITE BIOLOGIQUE

(30) Priority: 13.06.2000 US 211268 P; 30.05.2001 US 294531 P
(43) Date of publication of application: 12.11.2003
(73) Proprietor: GLAXO GROUP LIMITED, Greenford, Middlesex UB6 0NN (GB)
(72) Inventor: HAIZLIP, Jill Elaine, GlaxoSmithKline, Research Triangle Park, NC 27709 (US); IGNAR, Diane Michele, GlaxoSmithKline, Research Triangle Park, NC 27709 (US); JAYAWICKREME, Channa K., GlaxoSmithKline, Research Triangle Park, NC 27709 (US); KING, Holly Kay, GlaxoSmithKline, Research Triangle Park, NC 27708 (US); LIACOS, James Arthur, GlaxoSmithKline, Research Triangle Park, NC 27709 (US); YOUNG, Kirsten Marian, GlaxoSmithKline, Hertfordshire SG1 2NY (GB); RUAN, Jason J., GlaxoSmithKline, Raleigh Durham North Carolina 27709 (US); SAULS, Howard Ray, Jr., GlaxoSmithKline, Research Triangle Park, NC 27709 (US); SHAFFER, Joel Edward, GlaxoSmithKline, Research Triangle Park, NC 27709 (US)
(74) Representative: Giddings, Peter John
(86) International application number: PCT/US2001/019033
(87) International publication number: WO 2001/096597

(56) References cited:
- WO-A-00/17643
- WO-A-96/01426
- WO-A-96/30760
- US-A- 5 780 258
- US-A- 5 854 004
- US-A- 6 057 114

### SEQUENCE LISTING

<110> GLAXO GROUP LIMITED
<120> HIGH THROUGHPUT METHOD FOR SCREENING CANDIDATE COMPOUNDS FOR BIOLOGICAL ACTIVITY
<130> Docket No. PU3763WO
<140>
   <141>
<160> 3
<170> Patent In Ver. 2.1
<210> 1
   <211> 6
   <212> PRT
   <213> Artificial Sequence
<220>
   <223> Description of Artificial Sequence: MCR1 agonist peptide
<400> 1
   Ser Val His Phe Arg Trp
    1               5
<210> 2
   <211> 6
   <212> PRT
   <213> Artificial Sequence
<220>
   <223> Description of Artificial Sequence: MCR1 agonist peptide
<400> 2
   Val Ala His Phe Arg Tyr
    1               5
<210> 3
   <211> 8
<212> DNA
   <213> Artificial Sequence
<220>
   <223> Description of Artificial Sequence: cre motif
<400> 3
   tgacgtca 8

## Description

### Technical Field

The present invention pertains generally to a high throughput method for screening candidate compounds for biological activity. More particularly, the present invention pertains to a high throughput method for screening candidate compounds for biological activity that employs a substrate comprising the target of interest.

**Table of Abbreviations**

| | |
|---|---|
| λ - | wavelength |
| 7TM - | 7 transmembrane |
| AC - | adenylyl cyclase |
| BSA - | bovine serum albumin |
| cAMP - | cyclic AMP |
| CFM - | conditioned fibroblast (or frog) media |
| CHO - | Chinese hamster ovary |
| CRE(s) - | cAMP response element(s) |
| CRH - | corticotrophin releasing factor |
| CRHR1 - | corticotrophin releasing factor receptor |
| DMEM/F12 - | Dulbecco's modified Eagle's medium |
| DMSO - | dimethyl sulfoxide |
| EDTA - | ethylenediaminetetraacetic acid |
| ELISA - | enzyme-linked immunosorbent assay |
| FBS - | fetal bovine serum |
| GFP - | green fluorescent protein |
| GPCR - | G protein coupled receptor |
| GLP-1 - | glucagon-like peptide-1 |
| FLIPR - | fluorescence imaging plate reader |
| HDR - | high density replicator |
| HEK - | human embryonic kidney |
| HTS - | high throughput screening |
| L-15 - | Leibovitz-15 medium |
| LMP - | low melting point |
| MC1R - | melanocortin-1 receptor |
| MDS - | methods development software |
| Min. - | minute(s) |
| ml - | milliliter(s) |
| mRNA - | messenger RNA |
| αMSH - | alpha melanocyte stimulating hormone |
| [NDP]-αMSH- | 4-L-norleucine-7-D-phenylalanine-alpha melanocyte stimulating hormone |
| nl - | nanoliter(s) |
| PBS - | phosphate buffered saline |
| PD - | pigment dispersion |
| PLC - | phospholipase C |
| RT-PCR - | reverse transcription polymerase chain reaction |
| SOP - | standard operating procedure |
| Tcl - | tool command language |
| TPA - | phorbol 12-myristate 13-acetate |
| UV - | ultraviolet |
| WT - | wild type |

### Background Art

The advent of combinatorial chemistry techniques has enabled the synthesis of large numbers of compounds that have potential as therapeutic agents. However, assays that can rapidly and accurately identify those candidate compounds having pharmacological activity have lagged behind the pace of synthesis. As a result, many pharmaceutical companies possess vast libraries of candidate compounds that have not been screened for pharmacological activity.

A PIN tool system is a commercially available system that is typically used to transfer 96 or 384 samples from a reservoir to a receptacle. Currently, PIN tool technology is applied in DNA/RNA spotting on membranes. A representative PIN tool system, along with a description of the use of the device to transfer samples to a 384-well plate, is disclosed by Brant, "Multiplexed Nanoliter Transfer for High Throughput Drug Screening Using the BIOMEK^{®} 2000 and the High Density Replicating Tool", *Journal of Biomolecular Screening,* Vol. 2(2), (1997), published by Beckman Industries, Inc., Biotechnologies Development Center, Fullerton, California.

U.S. Patent No. 5,601,922 issued to Lerner et al. on February 11, 1997 (Bunsen Rush Laboratories, Inc. of Newton, Massachusetts) discloses peptide library formats and method relating thereto. The application of synthetic peptide library beads onto an agarose layer overlayed on cells to detect functional responses is disclosed.

U.S. Patent No. 5,976,813 issued November 2, 1999 to Beutel et al. (assignee Abbott Laboratories of Abbot Park, Illinois) discloses a continuous format high throughput screening assay. The disclosed assay employs at least one porous matrix upon which each of more than 96 samples of distinct samples of substances is deposited in the assay. In a cell-based embodiment of the assay, cells expressing a receptor of interest are grown or plated on a cell matrix. A porous matrix is prepared such that a labeled ligand for the receptor is dispensed onto or into the matrix. Test compounds or samples are then dispensed directly onto the ligand or cell matrix, or alternatively on or into another matrix. In this case, the sample matrix is brought in contact with the ligand matrix thereby allowing the sample to diffuse into the ligand matrix. After a suitable incubation period the ligand matrix is brought into contact with the cell matrix, preferably on the non-cell side of the matrix and the ligand and sample contact and react with the receptor by diffusion. Thus, the cell-based embodiment requires the preparation of a separate cell matrix.

Despite the disclosure of the foregoing documents, there remains substantial room for the development of improved high throughput screening methods for pharmacologically active candidate compounds, particularly for use with liquid samples and/or for use in a cell-based assay. A method for screening candidate compounds for pharmacological activity against a variety of targets thus represents a long felt and continuing need in the art.

### Summary of the Invention

A method for screening candidate compounds for an ability to modulate the biological activity of a target is disclosed. The method comprises providing a substrate comprising a target and an indicator, the substrate adapted for receiving a plurality of candidate compounds; contacting the substrate with a plurality of candidate compound samples in a manner wherein an identity of each candidate compound can be determined and wherein the candidate compounds in the candidate compound samples interact with the target in the substrate; detecting a signal, the signal produced by the indicator upon interaction between the target and a candidate compound; and identifying a candidate compound as a modulator of the biological activity of the target based upon an amount of signal produced.

In an alternative embodiment, a method for screening candidate compounds for an ability to modulate the biological activity of a target is disclosed. The method comprises providing a substrate comprising a target and an indicator, the substrate adapted for receiving a plurality of candidate compounds; contacting the substrate with a plurality of liquid candidate compound samples to form a predetermined pattern of candidate compound samples on the substrate, whereby the candidate compounds in the candidate compound samples interact with the target in the substrate within the predetermined pattern; detecting a signal within the predetermined pattern, the signal produced by the indicator upon interaction between the target and a candidate compound; and identifying a candidate compound as a modulator of the biological activity of the target based upon an amount of signal produced as compared to a control sample.

In an alternative embodiment, the method comprises: providing a substrate comprising a matrix, an indicator and a plurality of cells expressing the target, wherein the plurality of cells are suspended in the substrate, and
wherein the substrate is adapted for receiving a plurality of candidate compounds; contacting the substrate with a plurality of candidate compound samples in a manner wherein an identity of each candidate compound can be determined, and wherein the candidate compounds within the candidate compound samples interact with the target within the substrate; detecting a signal produced by the indicator upon interaction between the target and a candidate compound; and identifying a candidate compound as a modulator of the biological activity of the target based upon an amount of signal produced as compared to a control sample. In this embodiment, the cells preferably comprise mammalian cells.

Accordingly, it is an object of the present invention to provide a novel assay method for screening candidate compounds for an ability to modulate the biological activity of a target. The object is achieved in whole or in part by the present invention.

An object of the invention having been stated hereinabove, other objects will become evident as the description proceeds when taken in connection with the accompanying Drawings and Laboratory Examples as best described herein below.

### Brief Description of the Drawings

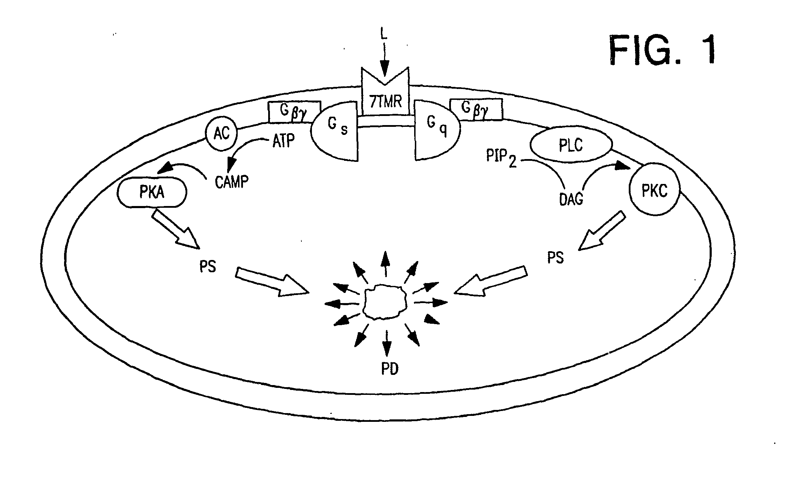

Figure 1 is a schematic drawing of 7TM receptor signal transduction in *Xenopus* melanophores.

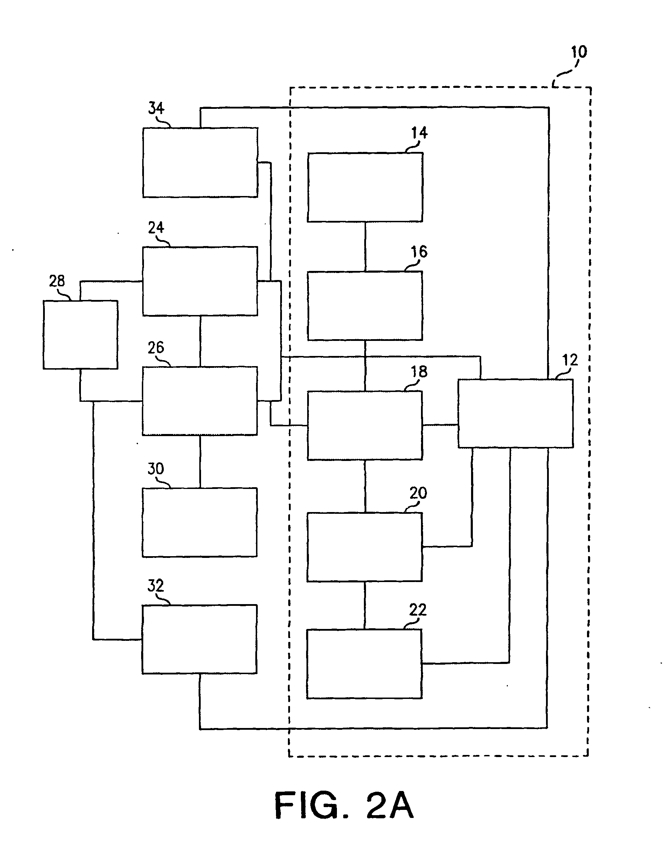

Figure 2A is a block diagram of a system suitable for use in the assay method of the present invention.

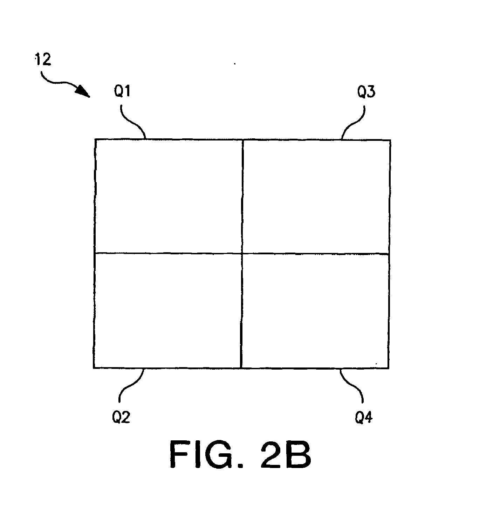

Figure 2B is a schematic drawing depicting an order for applying candidate compounds to a destination plate for use in the assay method of the present invention.

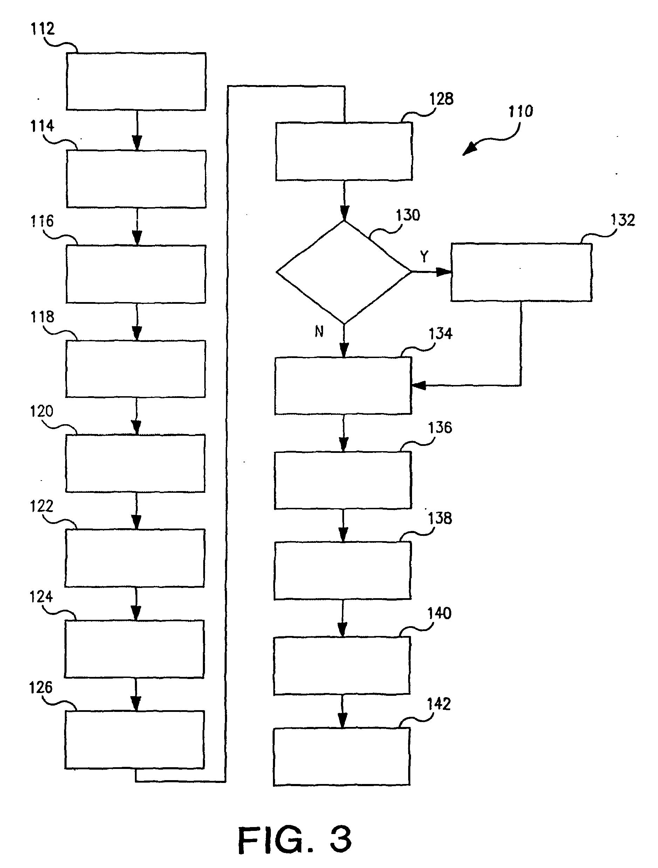

Figure 3 is a flow chart depicting steps for analyzing images generated in accordance with the assay method of the present invention.

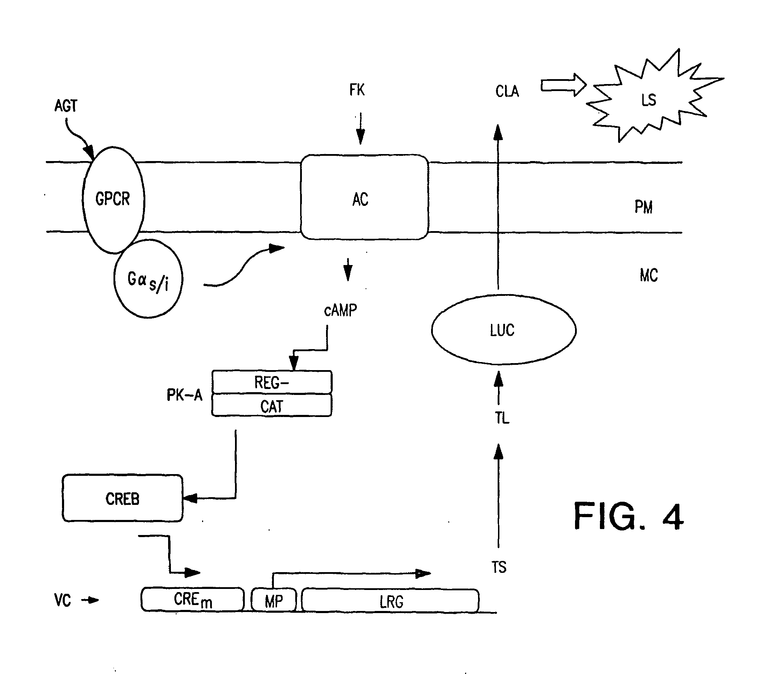

Figure 4 a schematic diagram of GPCR activation of CRE response elements driving transcription of luciferase.

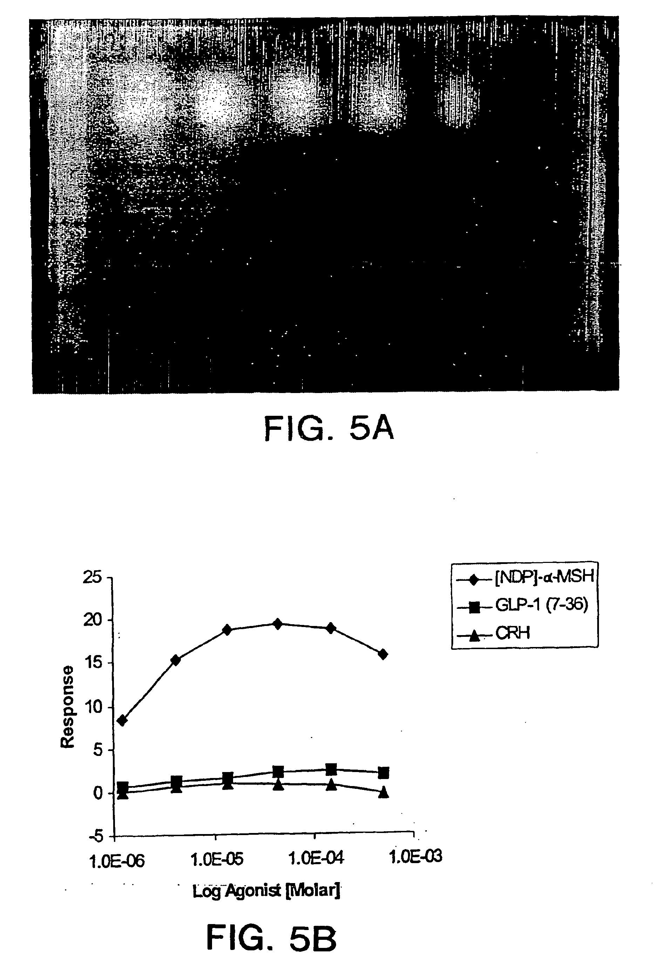

Figures 5A and 5B depict agarose lawns containing CHO-6xCRE-luc⁺ cells expressing MC1 receptor, which were treated with the concentrations of 4-L-norleucine-7-D-phenylalanine-alpha melanocyte stimulating hormone [NDP]-αMSH (top row in Figure 5A; ◆ in Figure 5B), glucagon-like peptide-1 (GLP-1) (7-36) (middle row in Figure 5A; ■ in Figure 5B), or corticotrophin releasing factor (CRH) (bottom row in Figure 5A; **▲** in Figure 5B) as described in Examples 7-10. Figure 5A shows the image and Figure 5B is a graphical representation of the concentration response curves for each peptide. The trays were incubated for 4 hours at 37°C before addition of LUCLITE PLUS^{™} reagent and subsequent imaging on the VIEWLUX^{®} camera. The response was calculated by subtracting the background z values from the z values for the area where peptide was delivered.

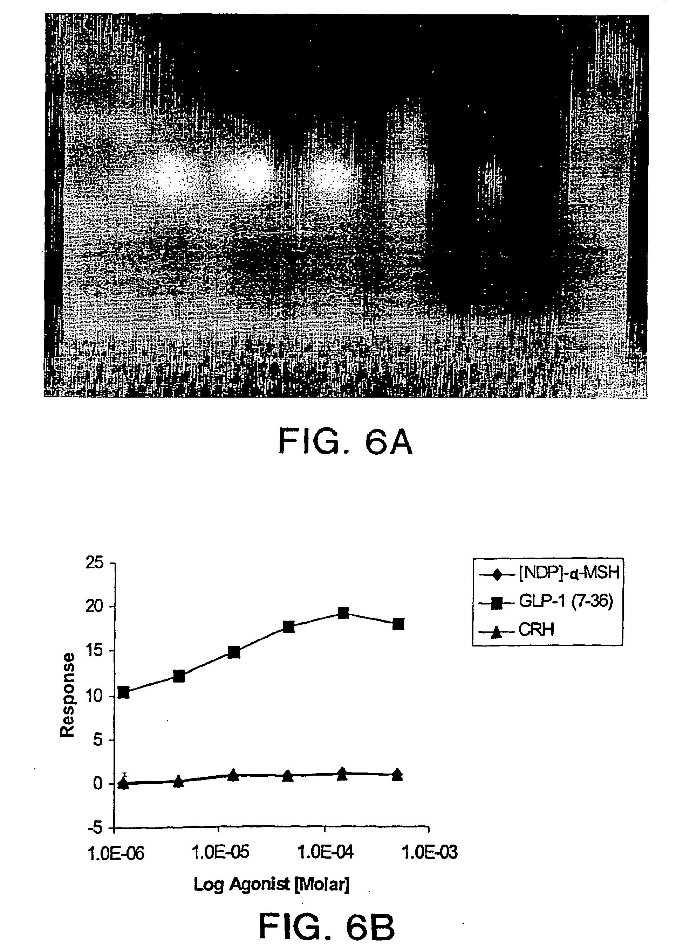

Figures 6A and 6B depict agarose lawns containing CHO-6×CRE-luc⁺ cells expressing glucagon-like peptide-1 (GLP-1) receptor, which were treated with the concentrations of [NDP]-αMSH top row in Figure 6A ; ◆ in Figure 6B), GLP-1 (7-36) (middle row in Figure 6A; ■ in Figure 6B), or CRH (bottom row in Figure 6A; **▲** in Figure 6B) as described in Examples 7-10. Figure 6A shows the image and Figure 6B is a graphical representation of the concentration response curves for each peptide. The trays were incubated for 4 hours at 37°C before addition LUCLITE PLUS^{™} reagent and subsequent imaging on the VIEWLUX^{®} camera. The response was calculated by subtracting the background z values from the z values for the area where peptide was delivered.

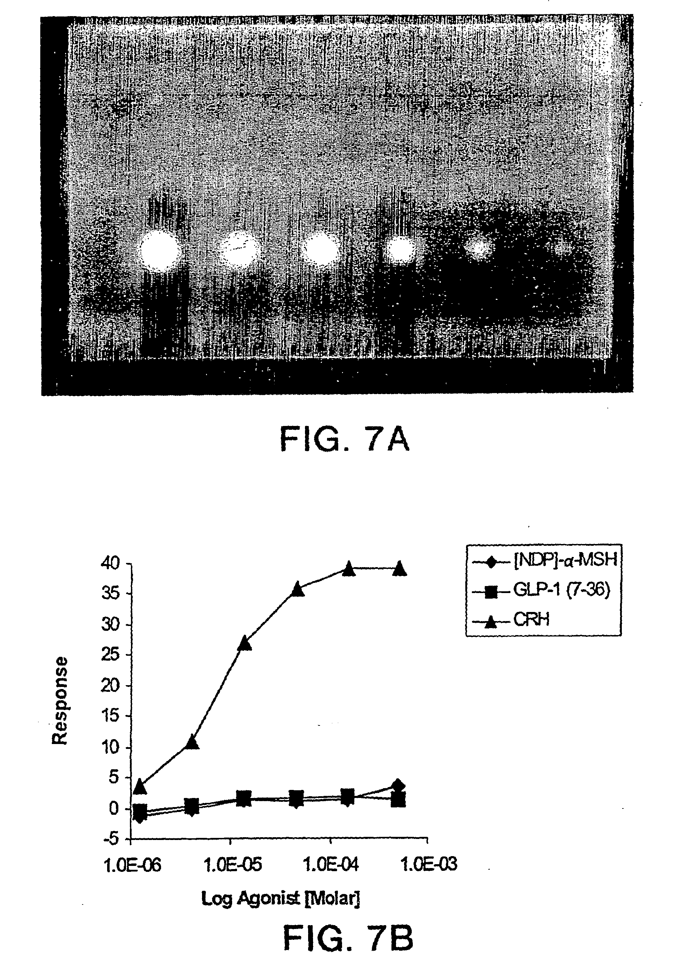

Figures 7A and 7B depict agarose lawns containing CHO-6xCRE-luc⁺ cells expressing corticotrophin releasing factor receptor (CRH1R), which were treated with the indicated concentrations of [NDP]-αMSH (top row in Figure 7A; ◆ in Figure 7B), GLP-1 (7-36) (middle row in Figure 7A; ■ in Figure 7B), or CRH (bottom row in Figure 7A; **▲** in Figure 7B) as described in Examples 7-10. Figure 7A shows the image and Figure 7B is a graphical representation of the concentration response curves for each peptide. The trays were incubated for 4 hours at 37°C before addition of LUCLITE PLUS^{™} reagent and subsequent imaging on the VIEWLUX^{®} camera. The response was calculated by subtracting the background z values from the z values for the area where peptide was delivered.

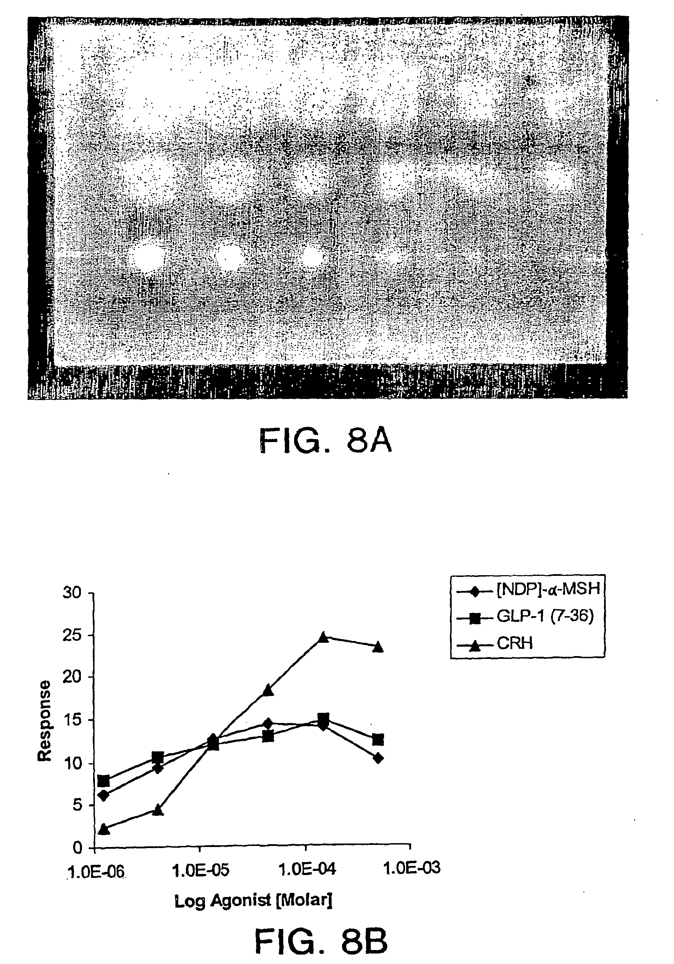

Figures 8A and 8B depict agarose lawns containing CHO-6xCRE-luc⁺ cells expressing MC1 receptor, GLP-1 receptor and CRH1R, which were mixed together and plated in a single lawn. The cells were treated with the concentrations of [NDP]-αMSH (top row in Figure. 8A; ◆ in Figure 8B), GLP-1 (7-36) (middle row in Figure 8A; ■ in Figure 8B), or CRH (bottom row in Figure 8A; ▲ in Figure 8B) as described in Examples 7-10. Figure 8A shows the image and Figure 8B is a graphical representation of the concentration response curves for each peptide. The trays were incubated for 4 hours at 37°C before addition of LUCLITE PLUS^{™} reagent and subsequent imaging on the VIEWLUX^{®} camera. The response was calculated by subtracting the background z values from the z values for the area where peptide was delivered.

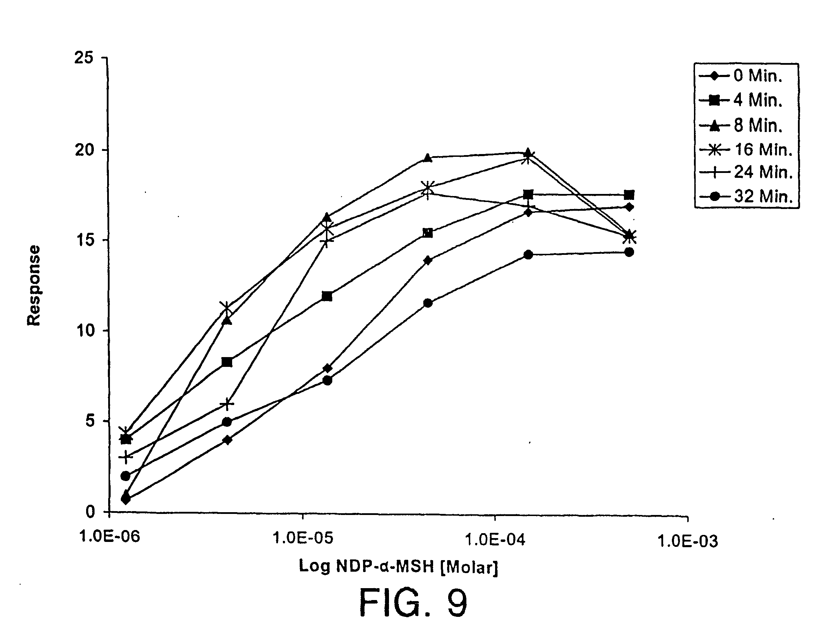

Figure 9 is a graphical representation of the concentration response curves for agarose lawns containing CHO-6xCRE-luc⁺ cells expressing MC1 receptor, which were exposed to ultraviolet light (λ = 365 nm) for the indicated times, i.e. **◆** = 0 Minutes (Min.), ■ = 4 Min., A= 8Min., X =16 Min., + = 24 Min., and • = 32 Min. The lawns were then treated with the [NDP]-αMSH as in Figures 5A and 5B. The trays were incubated for 4 hours at 37°C before addition of LUCLITE PLUS^{™} reagent and subsequent imaging on the VIEWLUX^{®} camera. The response was calculated by subtracting the background z values from the z values for the area where peptide was delivered.

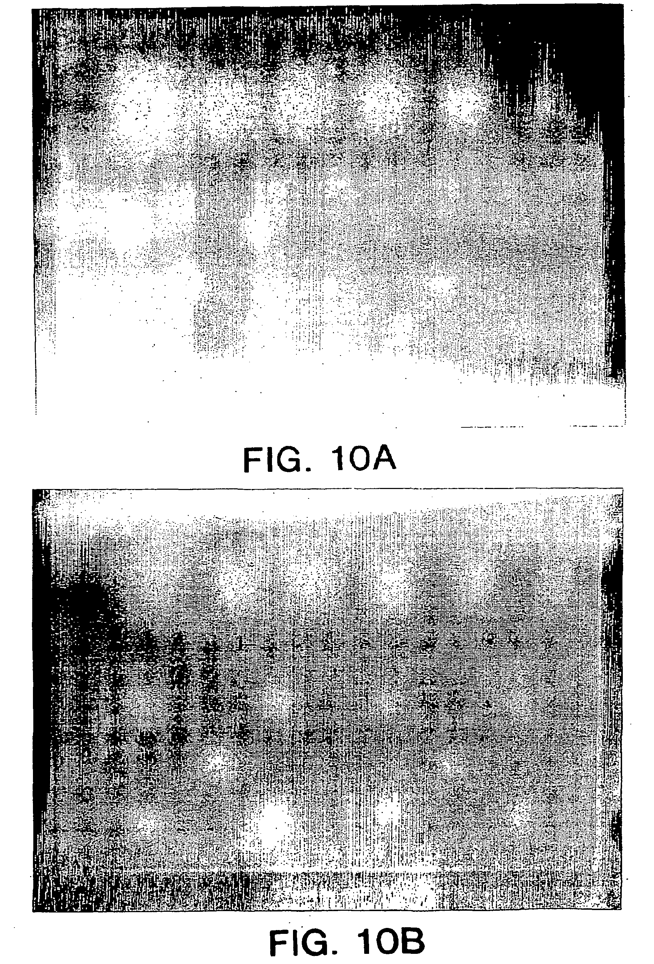

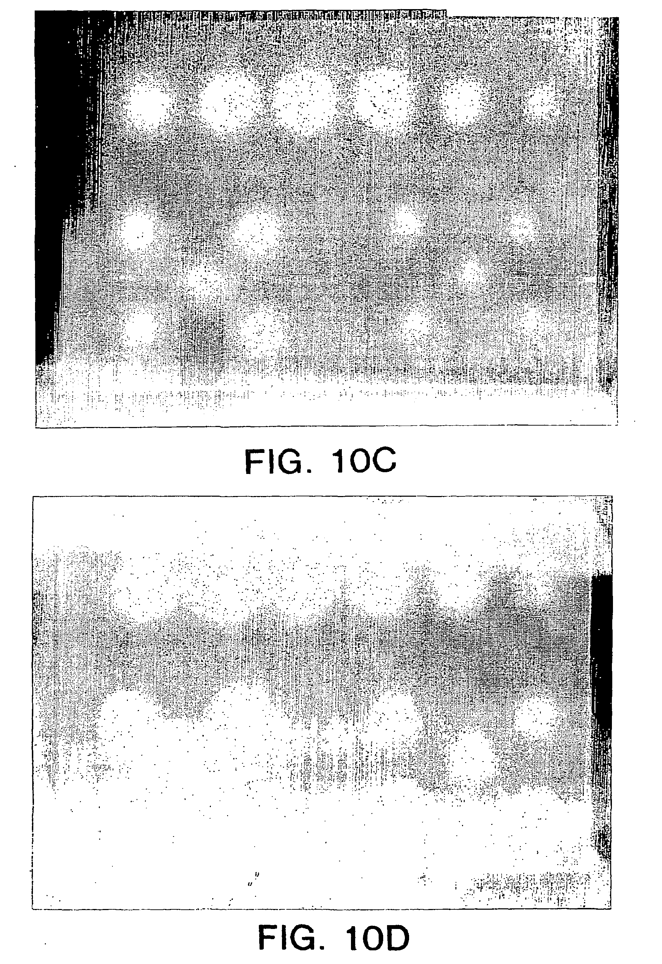

Figures 10A through 10E depict images of beads containing melanocortin-1 receptor (MC1R) agonist peptide 1 (5 beads on bottom left) or peptide 2 (5 beads on bottom right), which were inserted into agarose lawns containing CHO-6xCRE-luc⁺ cells expressing MC1R. The trays were then exposed to UV light (λ = 365 nm) for 0, 0.5, 1, 3, or 10 minutes (Figures 10A-10E, respectively). The lawn was treated with [NDP]-αMSH at the same concentrations as in Figure 5A (top row). The trays were incubated for 4 hours at 37°C before addition of LUCLITE PLUS^{™} reagent and subsequent imaging on the VIEWLUX^{®} camera.

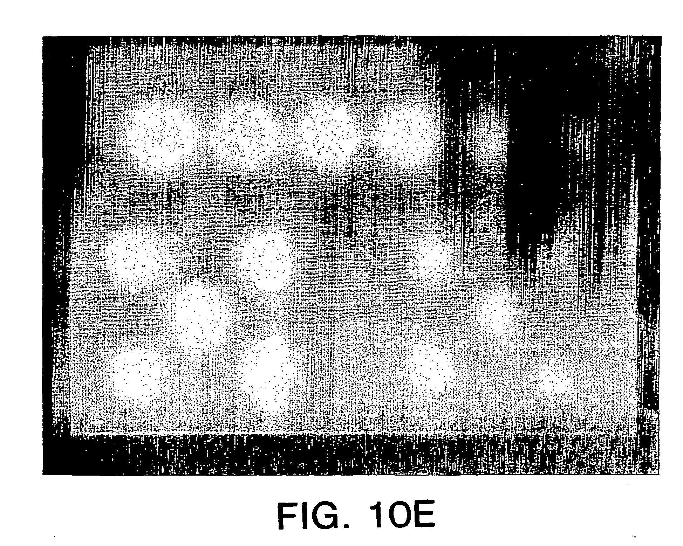

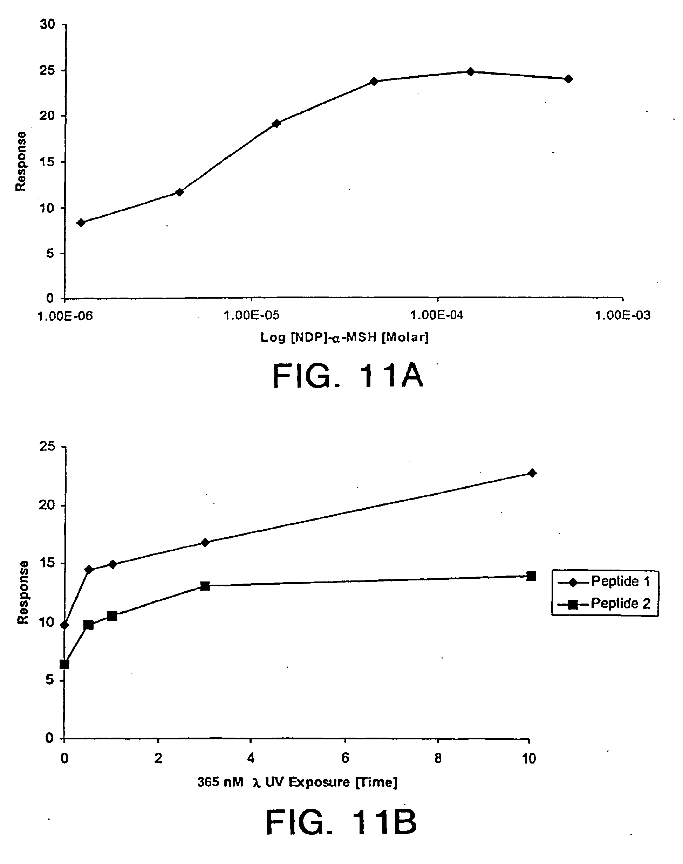

Figure 11A is a line graph representation of the [NDP]-α-MSH concentration curve from Figure 10E.

Figure 11B is a graphical representation of the average luminescence of the 5 beads described above for Figures 10A-10E. Each time point (◆ = peptide 1; ■ = peptide 2)) is shown. The response was calculated by subtracting the background z value for the lawn from the z value for each area to which the bead was applied.

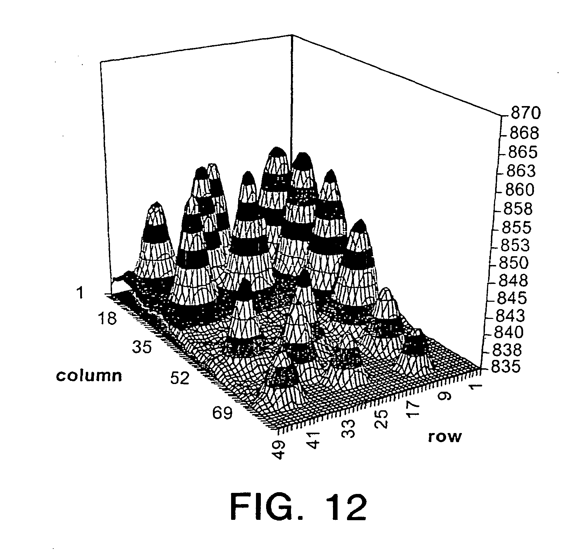

Figure 12 is a three-dimensional representation of the data from the 10-minute time point shown in Figure 10E. Z values are calculated as described herein below.

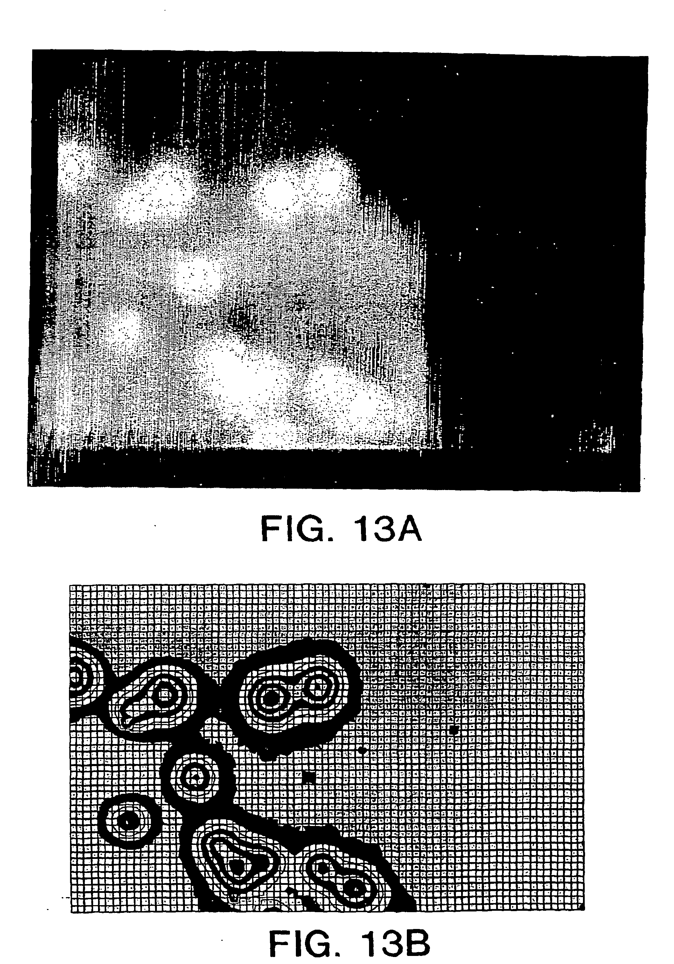

Figures 13A and 13B depict beads containing peptide 1 and 2, which were mixed with the agarose and cells before the lawn was poured. The hardened lawn containing the cells and beads was then exposed to UV light (λ = 365 nm) for 10 minutes. The trays were incubated for 4 hours at 37°C before addition of LUCLITE PLUS^{™} reagent and subsequent imaging on the VIEWLUX^{®} camera. The image is shown in Figure 13A and a contour graph is shown in Figure 13B.

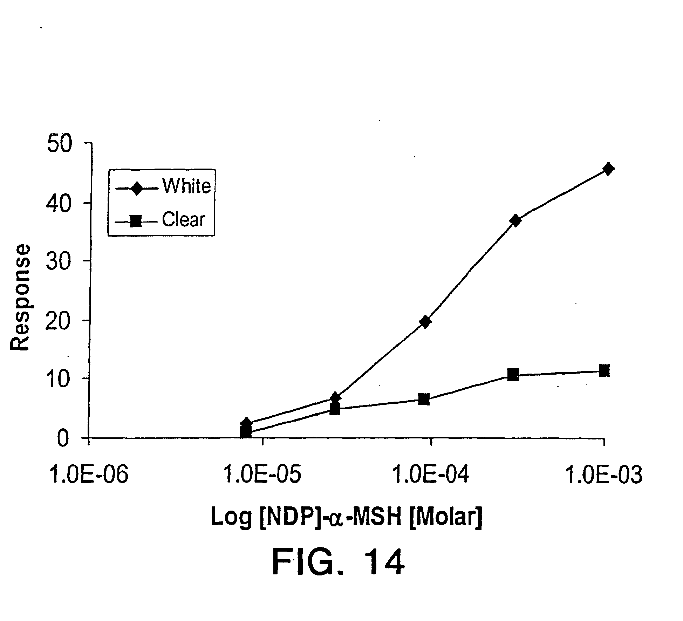

Figure 14 is a graphical representation of the concentration response curves for agarose lawns containing CHO-6xCRE-luc⁺ cells expressing MC1 receptor, which were plated in white (◆) or clear (■) OMNI PLATE^{®} trays and were treated with the concentrations of [NDP]-αMSH. The trays were incubated for 4 hours at 37°C before addition of LUCLITE PLUS^{™} reagent and subsequent imaging on the VIEWLUX^{®} camera. The response was calculated by subtracting the background z values from the z values for the area where peptide was delivered.

### Detailed Description of the Invention

A method for screening candidate compounds for an ability to modulate the biological activity of a target is disclosed. The method comprises providing a substrate comprising a target and an indicator, the substrate adapted for receiving a plurality of candidate compounds; contacting the substrate with a plurality of candidate compound samples in a manner wherein an identity of each candidate compound can be determined and wherein the candidate compounds in the candidate compound samples interact with the target in the substrate; detecting a signal, the signal produced by the indicator upon interaction between the target and a candidate compound; and identifying a candidate compound as a modulator of the biological activity of the target based upon an amount of signal produced as compared to a control sample.

In one alternative embodiment, the method comprises providing a substrate comprising a target and an indicator, the substrate adapted for receiving a plurality of candidate compounds; contacting the substrate with a plurality of liquid candidate compound samples to form a predetermined pattern of candidate compound samples on the substrate, whereby the candidate compounds in the candidate compound samples interact with the target in the substrate within the predetermined pattern; detecting a signal within the predetermined pattern, the signal produced by the indicator upon interaction between the target and a candidate compound; and identifying a candidate compound as a modulator of the biological activity of the target based upon an amount of signal produced as compared to a control sample.

In another alternative embodiment, the method comprises: providing a substrate comprising a matrix, an indicator and a plurality of cells expressing the target, wherein the plurality of cells are suspended in the substrate, and wherein the substrate is adapted for receiving a plurality of candidate compounds; contacting the substrate with a plurality of candidate compound samples in a manner wherein an identity of each candidate compound can be determined, and wherein the candidate compounds within the candidate compound samples interact with the target within the substrate; detecting a signal produced by the indicator upon interaction between the target and a candidate compound; and identifying a candidate compound as a modulator of the biological activity of the target based upon an amount of signal produced as compared to a control sample.

Following long-standing patent law convention, the terms "a" and "an" mean "one or more" when used in this application, including the claims.

### I. General Considerations

A reporter gene construct typically comprises a plasmid containing an inducible promoter element that controls the expression of a readily measurable enzyme activity or other protein (the reporter gene). The reporter gene itself typically has a unique activity or structure to enable it to be distinguished from other proteins that are present. The activity of the reporter gene product or the messenger RNA (mRNA) for the reporter gene can provide an indirect measurement of the transcriptional activity of the promoter sequence. The inducible promotor element is inactive or weakly active with respect to transcription until the transcription factor(s) that bind to and activate the promoter are themselves activated as a consequence of receptor-mediated signal transduction. The activated promoter drives the transcription of the reporter gene, which is then quantified by assaying the mRNA, the protein product itself through fluorescence or other properties, or most commonly, the enzymatic activity of the protein product. Examples of reporter genes include but are not limited to firefly luciferase, *Renilla* luciferase SEAP, β-galactosidase, aequorin, and green fluorescent protein (GFP). See Ignar and Rees, (2000) In Kenakin, T., Angus, J.A. (eds.), *The Pharmacology of Functional, Biochemical, and Recombinant Receptor Systems, Handbook of Experimental Pharmacology,* Springer-Verlag, Heidelberg, Vol. 148:391-412, for a review of reporter gene systems and their use in pharmacology.

Reporter gene assays are used for identification of ligands for targets of interest in high throughput screening (HTS) campaigns and for secondary pharmacological profiling of compounds. Target proteins include but are not limited to G protein-coupled receptors (GPCRs), nuclear receptors, kinases, proteases, ion channels, 1-transmembrane proteins and any other protein that modulates signal transduction pathways. Virtually any signaling event regulated by a target protein can be assessed using reporter gene systems. For instance, reporter genes have been used to assess the pharmacology of GPCRs that couple to Gαₛ, Gα_{q} or Gαᵢ (Stratowa et al., (1995a) *Curr. Opin. Biotechnol.* 6:574-581; Ignar and Rees, (2000) In Kenakin, T., Angus, J.A. (eds.), *The Pharmacology of Functional, Biochemical, and Recombinant Receptor Systems, Handbook of Experimental Pharmacology,* Springer-Verlag, Heidelberg, Vol. 148:391-412). Modulation of intracellular cyclic AMP (cAMP) by Gαₛ-linked receptors can be assessed using cAMP response elements (CREs) (Himmler et al., (1993) *Curr. Opin. Biotechnol.* 6:574-581). Gα_{q}-linked receptor-mediated regulation of protein kinase C-dependent signaling events can be measured using phorbol 12-myristate 13-acetate (TPA) response elements (Weyer et al., (1993) *Receptors Channels* 1:193-200; Stratowa et al. (1995b) *J. Recept. Signal Transduct. Res.* 15:617-630) and Gα_{q} or Gα₁-linked receptor modulation of mitogen-activated protein kinase regulation can be assessed through use of Gal4/elk-1 or Gal4/sap1a reporter systems. Bevan et al., (1998) *Neuroreport* 9:2703-2708.

In one embodiment, the present invention discloses for the first time a method that provides a substrate comprising a matrix and a plurality of cells expressing a target and a reporter gene. The plurality of cells is suspended in the substrate, and the substrate is adapted for receiving a plurality of candidate compounds. The substrate is contacted with a plurality of candidate compound samples in a manner wherein an identity of each candidate compound can be determined. The candidate compounds within the candidate compound samples interact with the target within the substrate, and upon interaction between the target and a candidate compound, a signal produced by the reporter gene is detected. A candidate compound is identified as a modulator of the biological activity of the target based upon an amount of signal produced, and the amount of signal is preferably compared to a control sample.

### II. Assay Methods

In a preferred embodiment, the assay method of the present invention pertains to a "well-less" approach to the rapid screening of a large number of candidate compounds for activity in the modulation of a target. The term "well-less" is meant to refer to the preparation and use of a substrate comprising the target or the indicator, or both, for receiving the candidate compounds to be screened. The substrate can vary in size, shape and composition, as disclosed herein. A predetermined pattern can be established on the substrate preferably through the use of an automated system, such as a PIN tool system. The use of a PIN tool facilitates the establishment of the predetermined pattern and, in turn, the predetermined pattern facilitates the detection of a response to an interaction between a candidate compound and a target.

The term "candidate compound" is meant to refer to any compound wherein the characterization of the compound's ability to modulate the biological activity of a target is desired. "Modulate" is intended to mean an increase, decrease, or other alteration of any or all biological activities or properties of a target.

Representative candidate compounds include xenobiotics such as drugs and other therapeutic agents, carcinogens and environmental pollutants, natural products, peptides and extracts, as well as endobiotics such as steroids, fatty acids and prostaglandins. Other examples of candidate compounds that can be investigated by the assay method of the present invention include, but are not restricted to, agonists and antagonists for cell membrane receptors and nuclear receptors, toxins and venoms, viral epitopes, hormones (e.g., opioid peptides, steroids, etc.), hormone receptors, peptides, enzymes, enzyme substrates, co-factors, lectins, sugars, oligonucleotides or nucleic acids, oligosaccharides, proteins, and monoclonal antibodies.

Liquid candidate compound samples are preferred for use in the assay method of the present invention. Significantly, as compared to standard well-based techniques, only a small volume of the liquid for each candidate compound is required for use in the present assay method. For example, a 10 nanoliter (nL) sample, as opposed to a 10 microliter (µL) sample as typically required in a well-based system, can be used in accordance with the present invention. Moreover, there are more candidate compounds currently available in liquid form. Thus, the assay method of the present invention can be used to screen many more compounds as compared to prior art systems, such as those based on well-format screens.

In another embodiment, candidate compounds are disposed on the substrate by mixing beads conjugated to candidate compounds with the substrate and cells. It is also possible to establish a peptide library using the bead format so that interaction of a peptide with a receptor can also be screened.

Candidate compounds are coded to beads in a conventional manner. To initiate the assay the beads are preferably exposed to a light source, preferably an ultraviolet (UV) light source, so as to photocleave the compounds from the beads and cause diffusion of the compounds into substrate. Preferably, the beads are loaded into an empty tray as a mixture with the cells in molten substrate (e.g., agarose) so that the beads are maintained at a single location. It is then possible to pick the bead and decode the compound if a signal associated with the bead is detected. Beads are loaded onto the substrate in a beads-per-square millimeter approach. It is thus typically possible to include approximately 100,000 beads in a single 1,536-format tray.

The substrate provided in accordance with the assay method of the present invention comprises a matrix component upon or within which candidate compounds can be deposited. The matrix component can comprise any suitable material. In a preferred embodiment, agarose is employed as a matrix component in the substrate. Optionally, a low melting agarose available from GibcoBRL of Gaithersburg, Maryland is used to prepare the substrate. Low melting point agarose solidifies at room temperature such that cells can be mixed into the agarose without a detrimental effect on viability. Low melting point agarose is also employed in some embodiments of the present invention to facilitate drying.

While agarose is a preferred matrix component of the substrate, other suitable matrix components, whether natural, synthetic or combinations thereof, as would be apparent to one of ordinary skill in the art after reviewing the disclosure of the present invention, can be used to make the substrate. For example, other biological matrix materials, including but not limited to collagen, laminin and the basement membrane derived biological cell culture substrate sold under the registered trademark MATRIGEL^{®} by Collaborative Biomedical Products, Inc. of Bedford, Massachusetts, can be used to make the substrate. Synthetic polymer substrate materials also can be used to make the substrate.

In a preferred embodiment variables associated with the preparation of the substrate are closely monitored. For example, size, solidification time, drying time and humidity are monitored in preparing and maintaining the substrate. Representative monitoring steps are disclosed in the Examples presented below. The substrate is also preferably formed or prepared on or in a solid support, such as a standard size or custom open format assay tray (for example, the top of or inside of a lid of a standard 1,536-well assay tray) to assure that the surface area of the substrate is sufficient to accept a predetermined pattern that accommodates the preferred 1,536 samples. A customized tray or other support can also be used, wherein the footprint of the tray is preferably large enough to accommodate 1,536 samples. Preferred outer dimensions for the footprint of a customized tray are 3.365 centimeters (cm) by 5.025 cm. The growth area for the customized tray is 94 square cm. The customized tray can be purchased from Nalge Nunc International of Rochester, New York and Naperville, Illinois under the registered trademark OMNI PLATE^{®}. Preferably, the solid support, e.g. OMNI PLATE^{®} tray, is colored to facilitate detection of the signal. More preferably, the color of the solid support is white.

In an alternative embodiment, 9,600 spots of liquid, including candidate compounds and controls, are established in the predetermined pattern on the substrate using an automated system as described herein. Thus, an aspect of the method of the present invention is to provide a substrate of a size sufficient to accommodate within the predetermined pattern on the substrate any desired multiplier of the number of pins available for use in transferring candidate compounds from a source plate or reservoir or of any desired multiplier of the number of candidates compounds available for transfer from a source plate or reservoir. The multiplier can range, for example, from 1 to 25, including preferably 2, 4, 8, 16, 20 and 25, e.g. 4 X 96 = 384, 4 X 384 =1,536; 4 x 1,536 = 6,144; and 25 X 384 = 9,600.

In a preferred embodiment, the substrate comprises a rectangle or square. However, any substrate shape suitable for a particular assay and as would be apparent to one of ordinary skill in the art after reviewing the disclosure presented herein can be implemented. Thus, for example, the substrate can comprise a triangular, circular, trapezoidal, oval or any other suitable geometrical shape. In the case of a circular or round substrate, a petri dish can be used to form the substrate.

In one embodiment of the present invention wherein cells are employed in the substrate, cells are poured onto an assay plate lid or other support. Optionally, the lid or support can be treated with a composition that improves adhesion, such as that sold under the trademark CELL-TAK^{®} by Collaborative Biomedical Products, Inc. of Bedford, Massachusetts. Cells should preferably completely cover the entire surface of the lid or support to form a lawn or layer of cells on the lid. The cell lawn is placed on a leveled surface and a matrix component, such as agarose as disclosed above, is then poured over the cell lawn, preferably after the cells have adhered to the surface. The substrate is allowed to set up for a time sufficient to solidify and dry the matrix so that candidate compounds can be loaded. In one embodiment, the substrate is allowed to set up for 45 minutes. Thus, the matrix component forms a layer over the cell lawn and, in accordance with one embodiment of the present invention, the substrate comprises two layers, a cell layer and a matrix layer. However, in an alternative embodiment, the cells can be mixed through the matrix component and in this case, the substrate comprises a single layer of cells and matrix.

In another embodiment, the cells can be mixed through the matrix component and in this case, the substrate comprises a single layer of cells and matrix. In the case of a suspension of cells in matrix, the suspension can be poured onto a support, such as a tray sold under the registered trademark OMNI PLATE^{®} by Nalge Nunc International. Preferably, the support or tray comprises a white plastic to enhance the ability to detect a signal.

In an embodiment of the present invention wherein cells are suspended throughout the substrate, it is preferred that the agarose remain solid at 37°C. Agarose concentrations ranging from 0.75% to 1.5%, including 1.0% and 1.25%, are preferably employed in the substrate. For example, cells can be diluted in a medium and then a 2X concentration of the desired end concentration of matrix medium is mixed with the cells in the medium. Thus, if a 10-milliliter sample of suspended cells is provided, 10 milliliters (mL) of 2% agarose is added to the cells, and 20 milliliters of 1% agarose comprising the cells is produced. Volumes of agarose preferably range from 5 mL to 50 mL, more preferably from about 5 mL to about 20 mL. More preferred volumes are 10 mL, 15 mL or 20 mL, with 12 mL comprising a most preferred volume. Cells can be present in total numbers ranging from about 0.5 to about 10 million per 1 mL of matrix (e.g. agarose). Preferably, cells are present in numbers ranging from about 1.5 to about 3.5 million per 1 mL of matrix (e.g. agarose). About 2.5 million cells per 1 mL of matrix (e.g. agarose), i.e. 25 million cells per 10 mL of matrix, are more preferred in terms of signal and cell culture effort.

The substrate also comprises a target. The term "target" is meant to refer to any chemical or biological entity having a biological activity for which the identification of an agent that modulates the biological activity is desirable. Multiple targets can be included in a substrate. Targets can be naturally occurring or manmade molecules. Also, they can be employed in their unaltered state or as aggregates with other species. Targets can be attached, covalently or non-covalently, to a binding member, either directly or via a specific binding substance. In a cell-based embodiment of the present invention, the target can be expressed by the cells and preferably, the cells are transfected to express the target. Indeed, cells can be transfected with multiple targets, and the screening of 1, 2, 3, 4 or more targets in a single effort is an aspect of the present invention. Additionally, different cells that express different targets can be employed in accordance with the present invention. Examples of targets which can be screened in the assay method of the present invention include, but are not restricted to, antibodies, cell membrane receptors, nuclear receptors, monoclonal antibodies and antisera reactive with specific antigenic determinants (such as on viruses, cells or other materials), drugs, oligonucleotides or nucleic acids, peptides, cofactors, lectins, sugars, polysaccharides, cells, cellular membranes, and organelles.

The substrate further comprises an indicator that identifies modulation of the biological activity of a target by a candidate compound. The term "indicator" is meant to refer to a chemical species or compound that is readily detectable using a standard detection technique, such as dark versus light detection, fluorescence or chemiluminescence spectrophotometry, scintillation spectroscopy, chromatography, liquid chromatography/mass spectroscopy (LC/MS), colorimetry, and the like. Representative indicator compounds thus include, but are not limited to, fluorogenic or fluorescent compounds, chemiluminescent compounds, colorimetric compounds, UV/VIS absorbing compounds, radionuclides and combinations thereof. A cell provided within the substrate can express the indicator, such as via a reporter gene, or the indicator can be provided in or added to the substrate. Representative reporter gene systems include a luciferase-CRE system; an NFAT-luciferase system; and/or a GAL4-ELK1 luciferase system.

Optionally, the indicator is produced by expression of the reporter gene and reaction of the reporter gene product with a signal development reagent (e.g. STEADY-GLO^{®} reagent available from Promega Corporation of Madison, Wisconsin or LUCLITE PLUS^{™} reagent described in the Laboratory Examples presented below) in the matrix. In this case, the method can comprise the steps of incubating the substrate for a predetermined time at a predetermined temperature; and contacting the substrate with a signal development reagent after the incubating step, whereby the indicator is produced by expression of the reporter gene and reaction of the reporter gene product with the signal development reagent. Representative "predetermined times" and "predetermined temperatures" are disclosed in the Examples presented below. By way of general summary, a representative predetermined time is that amount of time sufficient for expression of the reporter gene so that a sufficient concentration of reporter gene product is present in the substrate to react with the signal development reagent. Generally, a preferred predetermined time ranges from 2 hours to 6 hours, more preferably from about 4 to about 6 hours. A preferred predetermined temperature can comprise a temperature at which the cells in the substrate grow well and carry out cellular processes in a normal manner, e.g. 37°C for most cells, including mammalian cells. A preferred predetermined environment can comprise 95% oxygen (O₂) and 5% carbon dioxide (CO₂) in a humidified environment. Optionally, cells are lysed as necessary to permit and/or facilitate interact between the reporter gene product and the signal development reagent.

Any suitable cell can be used in a cell-based assay of the present invention..Representative cells include but are not limited to mammalian cells, such as Chinese hamster ovary (CHO) cells and human embryonic kidney (HEK) 293 cells; other vertebrate cells, such as melanophores from *Xenopus laevis;* insect cells; plant cells; bacterial cells; and fungal cells, such as yeast cells. Combinations of any of the foregoing cells can be used in a cell-based assay of the present invention, including but not limited to combinations of mammalian cells. Optionally, when combinations of different cells are employed, the different cells can express different targets in accordance with the present invention. Cell lines can be mixed together and dose response curves have been observed to be similar to one cell line plated alone.

In an embodiment of the present inventive method that employs candidate compounds conjugated to beads, CHO cells are preferred over melanophores in that CHO cells are believed to be less sensitive than melanophores to UV light, which is typically used to cleave the compounds from the beads. More UV light can thus be applied to provide more efficient cleavage. As discussed in the Laboratory Examples, UV light exposure does not seem to affect signal until approximately 16 minutes of exposure. Maximal signal is obtained by about 4 hours after agonist addition, (i.e. a preferred "predetermined time"). Signal read over a period of 18 hours in the VIEWLUX^{®} camera system remains completely stable for at least 3 hours and decreases over time after that.

Continuing with this embodiment of the present invention, the transfected CHO cells were diluted in a medium and then a 2X concentration of the desired end concentration of matrix medium is mixed with the cells in the medium. For example, if a 10-milliliter sample of suspended cells is provided, 10 milliliters of 2% agarose is added to the cells. Thus, 20 milliliters of 1% agarose comprising the cells is produced. This suspension was then poured onto a support, such as OMNI PLATE^{®} tray sold by Nalge Nunc International Inc. Preferably, the OMNI PLATE^{®} tray comprises a white plastic to enhance the ability to detect a signal.

When agarose is used as a matrix medium, it is preferred that the agarose remain solid at 37°C. After the agarose cools and hardens, the assay for modulator compounds of receptors of interest is initiated by:
1) adding compounds using a 96 or 384 well nail tool; or
2) adding compounds photolinked to beads to a tray in molten substrate mixed with cells and exposing the lawn to UV light to cleave compound from the bead.
Preferably, the cells are incubated in agarose for a predetermined time (preferably about four (4) - six (6) hours) after initiating the assay by addition of the compounds. After a predetermined time, again preferably about 4-6 hours, LUCLITE PLUS^{™} reagent (a stabilized glow system containing luciferin for detection of luciferase activity - available from Packard of Meridian, Connecticut) is poured on top of the lawn, allowing lysis of the cells in the agarose and penetration of luciferin substrate into the agarose. Thus, the indicator is produced by expression of the reporter gene and reaction of the reporter gene product with a signal development reagent (e.g. LUCLITE PLUS^{™} reagent) in the matrix. The signal has been observed to remain steady for at least 18 hours after addition of the substrate solution. Signal is detected using a VIEWLUX^{®} camera system available from Wallac Oy Corporation, Turku, Finland, or any CCD camera that is or can be cooled to just greater than about -20°C.

Thus, the signal can be detected by acquiring an image of the substrate. Optionally, the image of the substrate can also be acquired by focusing the signal from the substrate on a CCD camera chip via a telecentric lens. The detecting of the signal can further comprise digitizing the image, overlaying the image with a grid comprising a plurality of grid units and determining an average gray intensity value by scanning each of a predetermined number of grid units around the signal and averaging a gray intensity value of each grid unit in the predetermined number of grid units. The term "predetermined number" as used with respect to grid units refers to any suitable number of grid units (e.g. individual boxes or rectangles within the grid) upon which an average gray intensity value can be determined with a reasonable level of confidence. The average gray intensity value represents the z value for a given signal. Optionally, the method can further comprises recording x and y co-ordinates for the signal on the substrate. Suitable software packages for imaging luminescent or other light-based signals are available, and include the ROBOIMAGE^{™} software package (Glaxo SmithKline, Inc., Research Triangle Park, North Carolina, USA), as well as ADOBE^{®} PHOTOSHOP^{®} package with FOVIA PRO^{™} add in (Adobe Systems Incorporated, San Jose, California, USA) and IMAGE PRO^{®} PLUS package (Media Cybernetics, L.P., Silver Spring, Maryland, USA).

Other limits can optionally be imposed on signal characteristics to eliminate background noise and to facilitate identification and quantification of signal. Signal characteristics include diameter, intensity, circularity, concavity and volume, which are determined as described below. Volume and diameter of a signal can also be increased or decreased to facilitate correspondence between the number of rectangles available in the grid and the number of separate signals. For example, minimum volume and diameter can be established to minimize noise and the like. An operator can also manually add other signals upon visual inspection of the tray and upon defining a diameter in pixels.

By way of additional example, volume can be determined by slicing each signal in a serial of parallel chords one pixel apart. Volume is the summation of area under each chord. Diameter can be calculated as the square root of (4*area/Π). Intensity can be calculated as volume divided by area. Circularity can be calculated as 4π/(diameter X diameter). Concavity can be defined as maximum indention for the small, enclosed convex region.

When cells are employed, a variety of indicator systems can also be used. For example, *E. coli* can be transfected with a vector that encodes the luciferase protein. In this case, biological activity modulation is detected by a comparison of dark versus light in the predetermined pattern. Additionally, a FLIPR (fluorescence imaging plate reader, available from Molecular Devices of Sunnyvale, California) system can be used to detect calcium transfer via fluorescent dyes. In the calcium fluorescence system calcium that is released by a cell imparts change in fluorescence that can be detected. The cells themselves can also act as indicators, in that the detection of pigment aggregation/dispersion, cell outgrowth, cell replication, cell death and combinations thereof can be employed.

In a cell-based embodiment of the assay method of the present invention, cells can be transfected with multiple targets. The targets are chosen so that, to the extent possible, interactions between the targets are avoided. Additionally, untransfected control cells are included within the assay methods, so that apparently positive "hits" that are actually associated with endogenous molecules expressed by the cells can be excluded.

Thus, in a one embodiment, cells are suspended in the substrate and candidate compounds are disposed on the lawn using a PIN tool approach or by mixing beads conjugated to candidate compounds with cells and substrate and pouring the mixture into a tray or other suitable article in which a lawn can be formed. Preferably the cells, in addition to expressing a target receptor, also include a reporter gene system such that transcription of the reporter gene is operably associated with interaction of a candidate compound with the target receptor. It is possible to use different reporter systems that are associated with different receptors. This enables the screening of multiple receptors in a single effort. Preferred receptors include G protein-coupled receptors and it is thus possible to identify interactions with different receptors by coupling the different receptors with different reporter genes. In a more preferred embodiment, all 7TM receptors are screened in a single effort.

Preferably, antagonist compounds are screening in a bead-based embodiment of the present invention. In screening antagonist compounds, one plates the cells mixed with beads comprising antagonist compounds, and allows the lawn to harden. The lawn is exposed to UV light and after a desired or predetermined time (e.g. 15, 30, 45 or 60 minutes), a receptor agonist added to the surface of the lawn, such as (but not limited to) by pouring a solution of agonist on the lawn. Areas of substantially no signal, as can be determined, for example, using the z value calculations disclosed herein above, are observed if the candidate compound possesses antagonist activity.

The assay method of the present invention is applicable to any target (ion channel, nuclear receptor, kinase, protease, etc) in combination with a suitable reporter gene construct. Variance of the transcriptional response element driving the production of the report gene product will confer response to different types of targets. As noted above, the CRE-luciferase, GAL4/Elk-1-luciferase, and NF-AT luciferase systems, which respond to Gs, Gi/Gq or Gq-linked GPCRs, respectively, are representative reporter gene systems.

Referring now to Figure 1, melanophores from *Xenopus laevis* are preferred cells in that they can be transfected with a variety of target molecules (for example, a transmembrane 7TM target molecule **7TM**) while maintaining their pigment response characteristics. *Xenopus* melanophores respond to external stimuli by translocating melanin granuled within the cells. For example, pigment dispersion **PD** can be affected via activation of adenylyl cyclase **AC** or phospholipase C **PLC,** while pigment aggregation (not shown in Figure 1) results from the inhibition of adenylyl cyclase **AC.** Melanophores contain a wide range of Gα- and G_{βγ}-proteins, e.g., **Gₛ** and **G_{q}** (shown in Figure 1), and Gᵢ and Gₒ (not shown in Figure 1) which can couple with a foreign **7TM** receptor **7TM** for modulation via a ligand **L.**

In one embodiment of the present invention, *Xenopus* melanophores are poured onto an assay plate lid. Cells should preferably completely cover the entire surface of the lid to form a lawn or layer of cells on the lid. Then, the pigment in the cells is aggregated by adding melatonin to the cell lawn. The cell lawn is placed on a leveled surface, and agarose is then poured over the cell lawn and the plate is allowed to set for 45 minutes. Thus, the agarose forms a layer over the cell lawn and in accordance with one embodiment of the present invention, the substrate comprises two layers, a cell layer and an agarose layer.

Candidate compounds can then loaded onto the agarose layer using a PIN tool or other tool or using beads as described herein and an image of the plate is taken at time=0. Images are then recorded again at preselected interval over a specified time period, i.e. a predetermined or preselected time period for evaluation as disclosed herein below.

The assay method of the present invention can then further comprise flooding the substrate with a known ligand for a target expressed by the melanophores in the substrate in an effort to induce a change in pigment, i.e. pigment dispersion or pigment aggregation. Indeed, in a melanophore assay, both antagonist candidate compounds and agonist candidate compounds can cause either pigment dispersion or pigment aggregation, depending on the target being screened. Images are then recorded. These steps are taken to evaluate whether one of the candidate compounds modulates (i.e., inhibits or promotes, agonizes or antagonizes) the interaction between the known ligand and the target.

Since both states of intracellular pigment distribution (dispersion or aggregation) are easily detectable, numerous receptors, including 7TM receptors, can be studied by monitoring ligand-mediated pigment translocation. Therefore, a recombinant melanophore provides a powerful tool for drug discovery research and is preferred for use in the assay of the present invention. Preferred targets also couple to the same signaling system (melanosome dispersion or aggregation) in melanophores and therefore, if combined, activation of any one receptor will not interfere with the activation of another receptor by the same or different compound.

In other cell lines, antagonists are screened in a manner analogous to that set forth above with respect to melanophores. For example, the substrate can be contacted with a ligand for the target after loading the candidate compounds. The candidate compounds interact with the target in the presence of the ligand and a signal is detected. The signal is produced, or not produced, by the indicator upon blocking an interaction between the target and the ligand by the candidate compound. The candidate compound is thus identified by a candidate compound as an antagonist of the biological activity of the target based upon an amount of signal as compared to a control sample. For example, a darker area in a substrate comprising a luminescent indicator can indicate that a reporter gene that is expresses a luminescent gene product is not being expressed.

Referring now to Figure 4, a schematic diagram of GPCR activation of CRE response elements driving transcription of luciferase in a mammalian cell **MC** is presented. As shown in Figure 4, mammalian cell **MC** comprises plasma membrane **PM**, a G-coupled protein receptor **GPCR** bound within plasma membrane **PM,** adenylyl cyclase **AC** bound within plasma membrane **PM,** cAMP response element **CREB,** protein kinase A **PK-A,** and a vector construct **VC.** Vector construct **VC** comprises CRE motif **CREₘ** (TGACGTCA - SEQ ID NO:3), a minimal promoter **MP,** and a luciferase reporter gene **LRG.** In representative embodiment of an assay method of the present invention, a candidate compound agonist **AGT** contacts mammalian cell **MC** and interacts with a plasma membrane **PM**-bound G-coupled protein receptor **GPCR,** which in turn activates adenylyl cyclase **AC** via G protein subunit **Gαₛ.** Adenylyl cyclase **AC** interacts with cyclic AMP **cAMP**, which then interacts with protein kinase A **PK-A** and cyclic AMP response element **CREB** through its regulatory/catalyic cascade **REG-CAT** to activate transcription **TS** of vector construct **VC.** Luciferase **LUC** is thus produced by transcription **TS** and translation **TL** of luciferase reporter gene **LRG** in vector construct **VC.** After cell lysis and adminstration of signal development reagent (collectively referenced as **CLA** in Figure 4) as disclosed herein with respect to a preferred embodiment of the present invention, a light signal **LS** is detected.

Continuing with Figure 4, activation of **Gαᵢ** proteins causes inhibtion of adenylyl cyclase **AC.** Thus, in accordance with the present invention, one can assay for agonists of **Gαᵢ**-coupled receptors by looking at an inhibition of the stimulation of adenylyl cyclase **AC** by forskolin **FK.** In this embodiment, the substrate comprising the receptors is incubated with a candidate compound or compounds for a desired time (e.g. 30 minutes) and then forskolin **FK** is added.

A cell-free assay method is also provided in accordance with the present invention. By the term "cell free" it is meant that the substrate does not include cells and that the target, the indicator and the candidate compound are provided in a reaction medium within the substrate or are loaded onto the substrate in the predetermined pattern so that an interaction between the target, the candidate compound, the indicator or other provided reagent can be detected. A preferred example of a cell-free assay comprises an enzyme-linked immunosorbent assay (ELISA).

The term "predetermined pattern" is used herein for convenience to refer to any pattern that facilitates the localization of a signal produced by the interaction of a candidate compound with a target molecule to thereby characterize the candidate compound's ability to modulate the target.

In a preferred embodiment, the predetermined pattern comprises a rectangular pattern of "spots" or "touches" on the substrate. Rectangular patterns are preferred for convenience in that commercially available tools, such as a PIN tool, are amenable for use in establishing such a pattern. However, any predetermined pattern suitable for a particular assay and as would be apparent to one of ordinary skill in the art after reviewing the disclosure presented herein can be implemented. Thus, for example, the predetermined pattern can comprise a triangular, circular, trapezoidal, square, oval, or any other suitable geometrical pattern. Moreover, as described in detail below, all of the available locations for a "spot" or a "touch" need not be employed in a predetermined pattern of the present invention. Beads conjugated to candidate compounds can also be loaded in a predetermined pattern.

The predetermined pattern facilitates the localization of candidate compound "touches" or "spots" on the substrate so that the identity of a candidate compound at a particular "touch" or "spot" can be established. An image of the substrate having the predetermined pattern is recorded, and subtle changes in the target and the indicator are detected, preferably using the imaging steps disclosed hereinbelow. Preferably, in the case of a rectangular pattern, a rectangular grid pattern is imposed on the substrate in the imaging steps described herein. More preferably, the rectangular grid pattern further comprises 1,536 rectangles that correspond to and provide spot locations for depositing candidate compounds and pertinent control samples on the substrate, and provide the subsequent imaging of the same. Responses are detected in a timely manner so that any consequences of diffusion of the sample in the substrate is avoided.

In a preferred embodiment, 1,536 spots of liquid, including candidate compounds and controls, are established in the predetermined pattern on the substrate using an automated system as described herein. In an alternative embodiment, 9,600 spots of liquid, including candidate compounds and controls, are established in the predetermined pattern on the substrate using an automated system as described herein. Thus, an aspect of the method of the present invention is the accommodation within the predetermined pattern of any desired multiplier of the number of pins available for use in transferring candidate compounds from a source plate or reservoir or of any desired multiplier of the number of candidates compounds available for transfer from a source plate or reservoir. The multiplier can range, for example, from 1 to 25, including preferably 2, 4, 8, 16, 20 and 25, e.g. 4 X 96 = 384, 4 X 384 =1,536; 4 x 1,536 = 6,144; and 25 X 384 = 9,600.

In another alternative embodiment, 1,280 candidate compounds and 32 standards are spotted on the substrate. In this embodiment, eight (8) columns near the right or left edge of the substrate are reserved for the reference standards to facilitate comparison of the reference standards to the candidate compounds. Each column includes 32 possible spot locations. Hence, from a starting number of 1,536 spots, a reduction of 8 columns, each column comprising 32 spot locations, results in a capacity of 1,280 spot locations for candidate compounds. The reference standards are also preferably loaded onto the substrate so that at least one empty spot location is adjacent to each reference standard both vertically and horizontally to again facilitate comparison of the reference standards to the candidate compounds. The term "predetermined pattern" as used herein is thus also meant to encompass this approach for loading candidate compounds.

Manual systems can also be employed. A preferred manual system can be used with 384 samples, including candidate compounds and controls. In view of these numbers of samples and in view of the option of screening multiple targets at once, the high throughput aspects of the present assay method are believed to be apparent. Indeed, it is envisioned that over 300,000 compounds (with controls) per day can be screened using the method of the present invention, if desired.

The assay of the present invention is also preferred for screening biological targets that have been identified to be of therapeutic value, but have been screened against >100,000 molecules without identifying any leads nor many, if any, potential modulators.

### III. Assay System

Referring now to Figures 2A, 2B and 3, wherein like reference numerals refer to like parts throughout, a preferred system for use in the assay of the present invention is depicted, as is a flow chart associated with a preferred imaging technique employed in the system of the present invention.

Referring particularly to Figure 2A, the system of the present invention is referred to generally at **10.** In a preferred embodiment, system **10** comprises central processing unit (CPU) **12,** destination plates **14,** plate holders **16,** plate conveyor **18,** scanning stage **20,** camera **22,** arm **24,** PIN tool **26,** PIN tool storage area **28,** wash station **30,** fan **32,** and source plate **34.** Arm **24** comprises a PIN tool **26** having a plurality of pins, which is used to deposit candidate compounds onto the substrate in accordance with the methods of the present invention as described herein. Optionally, system **10** can further comprise an incubator and wash station **30** can further comprise a blotting station (not shown in Figure 2A).

Complete versions of system **10** that are suitable for use in the methods of the present invention are commercially available from Beckman Coulter, Inc. of Fullerton, California and are sold under the trademark SAGIAN^{™}. A suitable PIN tool system is commercially available from Beckman Coulter, Inc. of Fullerton, California, and is sold under the registered trademark BIOMEK^{®} 2000. PIN tool systems are preferred for use in the transfer of candidate compounds from 384-well source plates to a 1,536 spot capacity destination plate. A MULTIMEK^{™} 96 automated 96-channel pipettor (also available from Beckman Coulter, Inc. of Fullerton, California) can be used in the transfer of candidate compounds from 96-well source plates to a 384 spot capacity destination plate.

Continuing with reference to Figure 2A, CPU **12** calculates and transmits appropriate control signals for all components of PIN tool system **10.** A control protocol is established, for example, using a computer program file written in Tcl language and subsequently executed by control software employed by commercially available PIN tool systems, such as the BIOMEK^{®} BIOWORKS^{™} and BIOSCRIPT^{™} software employed in the BIOMEK^{®} 2000 system referenced herein. The Tcl program file contains the x, y and z coordinates along with other values for system movements in a machine language format. Thus, the Tcl program file provides variables that are input into CPU **12** and that are used by the control software to generate instructions that are executed by CPU **12** as discussed in the following description. Accordingly, references to inputs received, values and procedures established, and movements controlled by CPU **12** will be understood to refer to the Tcl program file as executed by and through the control software program or programs provided in CPU **12.** In a preferred embodiment, control signals are processed for four source plates of candidate compounds that are deposited into two assay plates for use in the assay of the present invention.

Continuing with reference to Figure 2A, locations in terms of x, y and z coordinates for arm **24** and PIN tool **26** are input into CPU **12** (as is an inclusive value for the amount of liquid dispensed and aspirated by PIN tool **26**). The number of source plates **34** is input into CPU **12,** as is the location of each source plate **34** in an x and y plane. A safe moving height (i.e., a height that facilitates clearance of structures within system **10**) for arms **24** and PIN tool **26** is established by the Tcl program file that is executed by CPU **12,** as is the height of the top of the source plates **34** and the aspiration depth for obtaining compound solution from a source plate. A typical aspiration depth for a standard 384-well source plate **34** is about 266 millimeters (mm).

Continuing with reference to Figure 2A, a location for a destination plate **14,** which supports the substrate and is carried on plate holder **16,** is established in the x and y plane, as is the top height and dispense depth for destination plate **14.** Destination plate **14** can be a destination plate or a control plate prepared as described herein. The dispensing depth for a plate that can accept 384 pins as compared to a plate that can accept 96 pins can vary. Further, for an embodiment that employs a lid of an assay plate as scaffolding for the substrate, a depth of about 267 mm is established.

Continuing with reference to Figure 2A, the location of wash stations **30** in the x and y plane are input into CPU **12.** The depth of each wash station **30** is assumed to be the same, and a z value for wash station **30** is thus also input into CPU **12.** A wash procedure is established, wherein PIN tool **26** is moved to wash stations **30** via arm **24** and control signals from CPU **12.** The wash procedure is established in CPU **12** by defining the location of a wash station **30** and providing the height of PIN tool **26** along with the location of the top and bottom of individual wash stations **30** and number of wash dips. The number of wash stations **30** to be employed and the number of up-and-down cycles (or dips) into a wash station **30** are also input into CPU **12.** The wash procedure is looped according to the number of times the pins of PIN tool 26 are to be washed. For example, three wash up-and-down cycles can be used and thus, the wash procedure is looped three times. PIN tool 26 is then moved by CPU 12 to fan 32 for drying using the x and y coordinates for fan 32 and a safe z coordinate path along which to move PIN tool 26.

Continuing with reference to Figure 2A, the procedure for transferring compounds from source plates 34 to destination plates 14 is established in CPU 12 by identifying the x and y locations of source plates 34 and of destination plates 14, along with the depth for depositing compounds onto destination plates 14. Transfers between source plates 34 and destination plates 14 are controlled by CPU 12 using the appropriate coordinates. Particularly, x and y coordinates for source plates 34 are used, along with a z safe coordinate for PIN tool 26. Additionally, the x and y coordinates are provided for destination plates 14, along with the appropriate depth of PIN tool 26 for dispensing compounds from source plates 34 onto destination plates **14.** In a preferred embodiment, the liquid candidate compound samples touch the surface of the substrate on destination plates 14, but PIN tool 26 does not contact the substrate.

Referring now to Figures 2A and 2B, in a preferred embodiment, the compound transfer protocol is repeated for quadrants Q1 - Q4 of a destination plate 14. Quadrants Q1 - Q4 are essentially square or rectangular and are of substantially the same size. Arm 24 is controlled by CPU 12 so that a first set of pin touches occurs in quadrant Q1; a second set of pin touches occurs in quadrant Q2, a third set of pin touches occurs in quadrant Q3 ; and a fourth set of pin touches occurs in quadrant Q4. In this embodiment, each set of pin touches comprises 384 individual pin touches so that 1,536 touches are provided within quadrants Q1-Q4. The touches of the individual pins thus provide the predetermined pattern. Arm 24 is also controlled by CPU 12 so that the pins of PIN tool 26 are washed at wash stations 30 then dried at fan 32 as described above between touches of quadrants Q1 - Q4 and so that the pins of PIN tool 26 communicate with source plates 34 after the washes but before each touch of destination plate 14. The compound transfer and washing procedures are repeated as necessary until all desired compounds are transferred from source plates **34** to destination plates **14.**

Continuing with reference to Figure 2A only, after completing compound transfers and final washes, PIN tool **26** is then returned by CPU **12** to a storage area **28** using x and y coordinates for PIN tool storage area **28,** along with a z safe coordinate for PIN tool **26.** Variables established for the x, y and z locations of source plates **34,** destination plates **14,** storage location **28,** wash stations **30,** fans **32** are unset in CPU **12** so that the system **10** can be used in a subsequent application.

Continuing with reference to Figure 2A, following compound transfer, destination plates **14** are immediately transferred to the scanning stage **20** via conveyor **18** or manually. Images are collected at pre-selected intervals (e.g., 5 minutes) using a CCD camera **22** (e.g., Model 14 available from Kodak of Rochester, New York) with the lens of approximately 60 mm focal length set on aperture F22 or other suitable setting. A representative camera that can also be used is a digital CCD camera sold under the trademark MICROMAX^{™} by SDR Clinical Technology - Princeton Instruments of Middle Cove, New South Wales, Australia. Another representative camera system is the VIEWLUX^{®} camera system available from Wallac Oy Corporation, Turku, Finland.

Continuing with reference to Figure 2A, images are recorded as an average of multiple acquisitions (e.g., five frames). Additional sets of plates **14** can be added to scanning stage **20** during image collection. Test and control destination plates **14** can be scanned in any order but are preferably alternately scanned in scanning stage **20.** Once images have been recorded up to a final (e.g., 30 minute) time point for the final set of test plates, the scanning schedule is stopped and all images are closed, such as by using a "close all" feature in a Microsoft Windows^{®} operating system. The recorded images are processed by CPU **12** as described hereinbelow.

Referring now to Figure 3, an imaging protocol for use in the system **10** of the present invention is referred to generally as **110.** In box **112,** images are collected for test and control plates at time=0, and then in box **114,** images are collected for test and control plates at a first pre-selected intervals (e.g., 5 minutes). Box **116** in Figure 3 depicts optimization of exposure time using 200/milliseconds (ms) as a starting point and using one test plate and one control plate to give z values of approximately 80% of max gray scale, also referred to herein as gray intensity or gray intensity scale for an 8 bit image. A z value refers to gray scale for an 8-bit image, which ranges from 0 to 255. Establishing a z value of approximately 80% of max gray scale for an 8 bit image for dark spots avoids overexposing the image to a "white out" and provides for a more readily detectable difference. Thus, where gray scale values can range from 0 to 255, the exposure is set to give z values of approximately 200. For light spots, z is established at approximately 20% above minimum gray scale.

Thus, the signal can be detected by acquiring an image of the substrate. Optionally, the image of the substrate can also be acquired by focusing the signal from the substrate a CCD camera chip via a telecentric lens. In this case the detecting of the signal further comprises digitizing the image, overlaying the image with a grid comprising a plurality of grid units and determining an average gray intensity value by scanning each of a predetermined number of grid units around the signal and averaging a gray intensity value of each grid unit in the predetermined number of grid units. The term "predetermined number" as used with respect to grid units refers to any suitable number of grid units (e.g. individual boxes or rectangles within the grid) upon which an average gray intensity value can be determined with a reasonable level of confidence. Optionally, the method can further comprises recording x and y co-ordinates for the signal on the substrate.

Continuing with Figure 3, box **118** provides for collecting images at additional pre-selected intervals (e.g., 5 minutes) over a pre-determined total time period (e.g., 30 minutes) and box **120** provides for saving all images by establishing a path and file name where the images are saved under an appropriate heading, such as "raw images". A file name format can be set up as follows: Mrx_date(mo/da/yr)A(first experiment that date) wherein x equals the number of targets screened on that date or wherein x equals the name of the target.

Continuing with reference to Figure 3, in box **122,** the zero minute image of a spot on a scanned test plate is subtracted from a 30-minute image to increase contrast. In box **124,** a grid of 1,536 rectangles is added to identify pin touches, and in box **126,** pin touches or "spots" are identified by enabling an operator command, preferably a "find spots" command. In box **128,** limits are imposed on spot characteristics to eliminate background noise and to facilitate identification and quantification of spots. Spot characteristics include diameter, intensity, circularity, concavity and volume, which are determined as described below. Volume and diameter of the spots can also be increased or decreased to facilitate correspondence between the number of rectangles available in the grid and the number of spots. For example, minimum volume and diameter can be established to minimize noise and the like. Box **130** provides that if any spots are missed by a "find spots" command (i.e. Yes, spots were missed - referred to as **Y** in Figure 3), the operator can manually add the spots in box **132** upon visual inspection of the plate and upon defining a diameter in pixels and adding a circle. If no spots are missed (referred to as **N** in Figure 3), imaging proceeds directly to box **134.**

Continuing with reference to Figure 3, in box **134,** images obtained from test and control plates are compared and in box **136,** positive "hits" are identified by imposing the grid comprising 1,536 rectangles over the image of the plate, rotating the image as necessary using the grid to line up pin touches, and setting the gray scale to improve contrast. The plates are imaged, gray scale adjusted and a threshold level for a positive hit for a modulator compound of the target within the test plate is set. Positive spots on test plates are analyzed while spots found on both test and control plates are deemed endogenous hits and are not further analyzed. In box **138,** positive "hits" are identified by correlating the position of the hit in the grid comprising 1,536 rectangles to the candidate compound deposited in that position. In box **140,** the images are saved into an appropriate file or can optionally be archived onto an optical disk or CD. In box **142,** image information is exported to appropriate word processing, spreadsheet or other software for organization and presentation.

In a preferred embodiment, volume is determined by slicing each spot in a serial of parallel chords one pixel apart. Volume is the summation of area under each chord. Diameter of a spot is calculated as the square root of (4*area/Π). Intensity is calculated as volume divided by area. Circularity is calculated as 4Π/(diameter X diameter). Concavity is defined as maximum indention for the small, enclosed convex region.

Any unused images are deleted, as are any greater than 30-minute images present in memory. Then, the camera/plate control and imaging procedure is activated as set forth above. Suitable WINDOWS^{®} operating system driven commands can be provided, such as "file", "open" and "image". The term "reference" can be enabled to select a reference image from memory.

### IV. Laboratory Examples

The following Laboratory Examples have been included to illustrate preferred modes of the invention. Certain aspects of the following Laboratory Examples are described in terms of techniques and procedures found or contemplated by the present inventors to work well in the practice of the invention. These Laboratory Examples are exemplified through the use of standard laboratory practices of the inventors. In light of the present disclosure and the general level of skill in the art, those of skill will appreciate that the following Laboratory Examples are intended to be representative only and that numerous changes, modifications and alterations can be employed without departing from the spirit and scope of the invention.

### Materials and Methods Used in Laboratory Examples 1-6 Vectors Encoding Targets

Obtain cDNA in pJG3.6 vector, described in Graminski, G. F., et al., *J. Biol. Chem.* (1993) 268:5957-5964, or other suitable vector) for each target that is to be transfected. If several receptors are being transfected simultaneously, optimal concentrations of stock solutions usually range from 5-10 mg/mL. Also, before a screening is started on a compound set, it is recommended to have enough target encoding cDNA on hand to complete the entire compound set (for a 300K compound set, typically a range of 10-20 mg, depending on the receptor).

### Other Materials and Reagents

CFM : Conditioned Fibroblast (or Frog) Media.

0.7X Trypsin/EDTA : Gibco BRL Cat # 25300-054, purchased as 1X stock.

0.7X PBS, pH 7.3: Prepared as 10X stock as: 80g/L NaCl, 2 g/L KH₂PO₄, 11.5 g/L Na₂HPO₄, 2.0 g/L KCl.

0.7X electroporation grade PBS, pH 7.0: same as PBS above except pH adjusted to pH 7.0.

0.7X L15/0.1 % BSA: Cat# B2799, Sigma/Aldrich Chemical Co. of St. Louis, Missouri.

DMSO: Cat# D8779, Sigma/Aldrich Chemical Co. of St. Louis, Missouri.

Melatonin : Cat # M5250, Sigma/Aldrich Chemical Co. of St. Louis, Missouri.

800 mL Electroporation Cuvettes: Model # 58017-900, VWR Scientific Products of West Chester, Pennsylvania.

225-cm³ tissue culture flask: Model # 3000, Costar of Oneonta, New York.

15 mL and 50 mL conical centrifuge tubes

Electroporator. ELECTRO CELL MANIPULATOR^{™}, Model BT 600, BTX of San Diego, California.

Centrifuge: SORVALL RT6000D^{™}, Kendro Laboratory Products, Newton, Connecticut.

SEAPLAQUE^{®} agarose: Cat# 50101, FMC Corporation of Philadelphia, Pennsylvania.

Lids (for assay plate sold under the registered trademark OMNI PLATE^{®}): Ref# SPO-161181, Nalge Nunc International of Rochester, New York and Naperville, Illinois

L15 w/o BSA: Cat# L-5520, Sigma Chemical Co. of St. Louis, Missouri.

### Example 1

### Electroporation of Melanophores for Transient Receptor Expression (DAY 01)

One to two hours prior to trypsinization, place 40 mL of fresh conditioned fibroblast (or frog) medium (CFM) onto 225 cm³ flasks of melanophores to be transfected. Wash each flask with 5 mL 0.7X PBS, pH 7.4, and aspirate. Add 5 mL 0.7X Trypsin/EDTA to each flask and let sit 3-5 minutes. Shake flask to dislodge cells and add 10 mL CFM to each flask.

Transfer contents of each flask into 50 mL conical centrifuge tubes. Spin 900 rpm for 5 min at RT. Aspirate media and pool pellets into one 50 mL conical centrifuge tube using CFM. Count cells to obtain a given concentration. Spin tube at 900 rpm for 5 minutes. Aspirate CFM and resuspend in 40 mL 0.7X PBS, pH 7.4. Spin 900 rpm for 5 minutes. Aspirate PBS and resuspend pellet in cold electroporation grade 0.7X PBS, pH 7.0 to a final concentration of 15 X 10⁶ cells/mL. Add 800 µL resuspended cells to cold microfuge tube containing mixture of cDNA. Incubate on ice for 20 min, mixing gently twice during incubation.

Transfer cDNA/cell solution to cold a 800 µL electroporation cuvette (w/o introducing bubbles). Electroporate at 500V, 625 mF, 720 ohms. Using a Pasteur pipette, immediately transfer electroporated cells to 30-40 mL of CFM. Place cells into 225 cm³ flasks (2 cuvettes per flask) in a final volume of 45 mL CFM. Place cells on flat bench for -3 hours. Aspirate media and replace with fresh CFM. Place cells in incubator for -24 hours.

### Example 2

### Preparation of Cell Plates (DAY 02)

Wash each flask with 5 mL 0.7X PBS, pH 7.4, and aspirate. Add 5 mL 0.7X Trypsin/EDTA to each flask and let sit 2-3 minutes. Shake flask to dislodge cells and add 10 mL CFM to each flask. Transfer contents of each flask into 50 mL conical centrifuge tubes. Spin at 900 rpm for 5 min at room temperature. Aspirate media and pool pellets into one 50 mL conical centrifuge tube using CFM. Count cells. Remove enough cells to make one 96 well plate (for quality control) at 30,000 cells per 100 µL per well. Prepare screen plates as described below.

Divide remaining cells among 50 mL conical tubes such that there are about 1-10 X 10⁶ cells in 27 mL CFM per tube. Mix the tubes by inversion but avoid creation of bubbles if possible. Gently pour the cells from one tube into a assay plate lid (cells should preferably completely cover the entire surface of the lid). Place the next lid to be filled on top of the lid just filled.

Repeat the above procedure until all tubes are empty. Place an empty lid on top of the last lid filled. Repeat the above steps using control cells (e.g., PTH1-3-30 cells), making the same number of assay plate lids and one 96 well plate (for quality control). Allow the lids and plates to sit on a flat bench at ambient temperatures (e.g., 25°C) for ~2 hours. Place lids and 96 well plates in an airtight container and place in incubator for overnight (about 15-24 hours).

### Example 3

### Quality Control Plates (DAY 03)

Prepare 50 mL of melanophore assay buffer containing 5nM Melatonin. Serial dilute peptides (natural ligands for receptors being screened) as follows: Transfer 2 µL aliquots of 100 µM stocks of each peptide to a separate 1.5 mL microcentrifuge tube. Add 998 µL melanophore assay buffer/5nM melatonin to each tube and vortex. Transfer 20 µL of each to separate wells of a 96 well round bottom plate by placing the first peptide into well A1 of the 96-well plate, second peptide into A2, etc... Add 180 µL melanophore assay buffer/5nM melatonin to these same wells. Add 137 µL to wells B1-B8, B2-B8, etc..., as far over on the 96-well plate as done for the 20 µL transfers above. Mix row A. Transfer 63 µL from row A to row B and mix. Transfer 63 µL from row B to row C and mix. Continue this serial dilution all the way down the plate.

The working concentrations (2X) when the above dilutions are completed are as follows:

| | | | |
|---|---|---|---|
| Row A | 20 nM | Row E | 200 pM |
| Row B | 632 nM | Row F | 63.2pM |
| Row C | 2 nM | Row G | 20 pM |
| Row D | 632 pM | Row H | 6.32 pM |

Aspirate CFM from control 96-well plate. Add 50 µL 0.7 L15/0.1 % BSA buffer containing 5 nM melatonin. Let plates sit 1-2 hours at room temperature. Read plates on SLT SPECTRA^{®} plate reader (available from Kollmorgen Corporation of Waltham, Massachusetts). Add 50 µL from serial dilution plate to each well. Incubate 1 hour. Read plate on SLT SPECTRA^{®} plate reader.

### Example 4

### Preparation of Cell Plates for Receiving Compounds (DAY 03)

Weigh out 1.25 g of SEAPLAQUE^{®} agarose into a 125 mL heavy walled bottle. Add 100 mL of L15 (Leibovitz) medium w/o BSA and heat in a microwave oven, uncapped at a LOW setting for about 15-18 min. The final solution should show complete dissolution of the agarose as a clear solution. Place this in a water bath pre-heated to 42°C. Place 2 or 4 mL of agarose into 50 mL polypropylene tubes and keep them in the water bath at 42°C. The number of tubes should equal the number of plates to be prepared.

Make up 200 mL of room temperature 0.7X L15/0.1% BSA containing 18 nM melatonin. Carefully pour off the media on the lids prepared above (one at a time), remove excess media and place the lid on a level surface. Transfer 10 mL of the 0.7X L15/0.1% BSA/18nM melatonin to a one of the 50 mL tubes containing 2 mL agarose with a pipette (preferred method) or 8 mL of the 0.7X L15/0.1 % BSA/18 nM melatonin to one of the 50 mL tubes containing 4 mL agarose. Use the pipette as a stirring rod while dispensing the 0.7X L15/0.1 % BSA/18nM melatonin.

Immediately and carefully pour the 12 mL mixture onto a assay plate lid ensuring that entire bottom of the lid is covered. Pop any bubbles with a dry needle. Repeat the above steps for each plate. Let assay plate lids sit at room temperature for 45 minutes. After 45 minutes, place the assay plate lids in a 95% humidified incubator (24°C) for storage until time for compound addition. These plates should be functional up to 12 hours.

### Example 5

### Preparation of Candidate Compound Source Plates

For each lid made containing transfected cells, remove from the freezer four 384-well source plates containing liquid candidate compound samples. Remove lids and any seal that may be on the plates. Place plates in stacks of four (plates should be kept in order using, for example, order of execution and plate numbers). Prepare sufficient 1µM αMSH in melanophore assay buffer to allow for 10 µL/well into 2 columns of every fourth drug plate. These columns provide control samples for the test plates.

The plates are marked by pipetting 10 µL of the above-referenced 1 µM αMSH solution into columns 22 and 24 of all the 384-well plates on top of each stack. Mark the end of each plate that received the 1 µM αMSH with an S (standard). The plates can also be marked by pipetting 10 µL of the above-noted 1µM αMSH solution into columns 21 and 23 of all the 384 well plates on bottom of each stack, and marking the end of each plate that received the αMSH with an S. One plate in every stack of four should now contain αMSH. Make sure the order of the plates does not change at any time.

### Example 6

### Compound Transfer and Assay

The Beckman BIOMEK^{®} 2000 System is used to transfer test compounds from 4 different 384-well source plates onto to 2 melanophore-containing substrates using the BIOMEK^{®} High-Density Replicating (HDR) PIN tool (Beckman part number 267618 -384 HDR). The HDR PIN tool contains 384 freefloating pins, which are either Posts or Nails. Posts transfer approximately 50-70 nL of each compound; nails transfer 10-30 nL. The program for compound transfer is written in Tool Command Language (Tcl), which provides the x, y and z coordinates for the x, y and z movements of the arm of the Beckman BIOMEK^{®} system. All plates are positioned to the back right of the plate holder.

Wash stations are loaded with 50 mL DMSO, 50 mL water and 60 mL ethanol to rinse pins thoroughly. The wash station also includes a blotting station containing a double thickness of paper towel placed inside a reservoir. All plates are positioned back and to the right of the plate holder. Transfers onto a test plate are in the order (See also Figure 2B):

| | |
|---|---|
| 1 | 3 |
| 2 | 4 |

For Gs/Gq agonists of the 7TM receptors expressed by the melanophores, darker spots develop on the gray cell lawn over time, directly below the HDR pin touches. Images are collected at 5-minute intervals using a CCD camera scanning stage setup (image station) as described herein above. Image collection and analysis are carried out according to the scanning stage layout of Table 1.

**Table 1**

| Scanning Stage Layout | | |
|---|---|---|
| A1 Test Plate 1 | A2 Control Plate 1 | A3 Test Plate 2 |
| B1 Control Plate 2 | B2 Test Plate 3 | B3 Control Plate 3 |
| C1 Test Plate 4 | C2 Control Plate 4 | C3 Test Plate 5 |
| D1 Control Plate 5 | D2 Test Plate 6 | D3 Control Plate 6 |

Once images are recorded up to a 30-minute timepoint for the final set of test plates, the schedule was stopped and all images were closed using the "Close All" command in the imaging system.

### Examples 7-10

### Mammalian Lawn Reporter Assays

Chinese hamster ovary (CHO) cells were transfected with a 6xCRE-luciferase⁺reporter gene. A stable host cell line was selected, into which cloned human receptors were transfected. Stable cell lines expressing these receptors were selected for screening purposes.

### Materials Employed in Examples 7-10

T-225 tissue culture flasks (#3001) were obtained from Coming Costar of Cambridge, Massachusetts. DMEM/F12 (#311039-021), Fetal Bovine Serum (FBS) (#16140-071), L-glutamine (#25030-081), 0.05% trypsin (#25300-054), Dulbecco's phosphate-buffered saline (PBS) (#4190-144) and LMP Agarose (#5517UB) were purchased from Gibco BRL of Gaithersburg, Maryland. EX-CELL^{™} 301 Medium (#14331-79P) was from JRH BioSciences of Lenexa, Kansas. LUCLlTE PLUS^{™} Luciferase Reporter Gene Assay Kit (#6016965) was purchased from Packard Instrument. Company of Meriden, Connecticut. Chinese hamster ovary (CHO-K1) cells were obtained from the American Type Culture Collection of Manassas, Virginia. Human GLP-1 (7-36) (#6795) and human CRF peptide (1-41) (#2435) were obtained from Bachem of King of Prussia, Pennsylvania. 4-L-norleucine-7-D-phenylalanine-alpha melanocyte stimulating hormone ([NDP]-αMSH, #8764) was obtained from Sigma Chemical Co. of St. Louis, Missouri. Clear and opaque white OMNI PLATE^{®} trays (altered footprint to accommodate HDR pin tool as disclosed herein above) were purchased from Nalge Nunc International Inc. A high-density replicating (HDR) tool, which has ninety-six 0.015-inch diameter transfer pins (#609089), was purchased from Beckman of Fullerton, California. UV lamp controlled by an AB-M H-1000 power supply box and an AB-M exposure control was purchased from ABM Inc. (San Jose, California). VIEWLUX^{®} camera was from Wallac Oy Corporation, Turku, Finland.

### Example 7

### Multiple Receptor Assay

Cell lines expressing both a reporter gene and a G protein-coupled receptor (GPCR) were assayed for response to agonist in an agarose lawn format. A cell line expressing a GPCR was plated by itself, and several cell lines expressing different GPCRs were mixed together to evaluate whether agonists for several receptors can be assayed simultaneously.

A reporter gene host cell line stably expressing a reporter construct comprising 6 cyclic AMP response elements (CREs) driving the expression of luciferase (6xCRE-luciferase⁺) was generated as described by Goetz et al., (2000) *J. Biomolec. Screening* 5:377-384. The full-length cDNAs for melanocortin-1 receptor (MC1R) (GenBank Accession # X65634) and corticotrophin releasing factor receptor (CRHR1) (GenBank Accession # X72304) were obtained by reverse transcription polymerase chain reaction (RT-PCR) and were subcloned into the expression vector, pCDNA3 (Invitrogen, Carlsbad, California). The full-length cDNA for human glucagon-like peptide-1 (GLP-1) receptor (GenBank Accession # U10037) was provided by Dr. Joseph Dillon (Tufts University) and was subcloned into pCDNA3. Stable CHO-6xCRE-luc⁺ cell lines expressing MC1R, CRHR1 or GLP-1 receptors were generated via electroporation as described by Goetz et al., (2000) *J. Biomolec. Screening* 5:377-384. The stable cell lines were propagated in T-225 tissue culture flasks in complete medium (DMEM/F12 containing 5% FBS and 2 mM L-glutamine).

Three days before assay, the cells were harvested using 0.05% trypsin, washed and resuspended in EX-CELL^{™} 301 medium containing 5% FBS and 2 mM glutamine at a concentration between 1 to 3 X10⁵ cells/ml. The cell suspension was placed in spin flasks and was propagated in suspension at 37°C, 5% CO₂ in a tissue culture incubator. Eighteen hours before assay, cells were removed from suspension by centrifugation and were resuspended in serum-free EX-CELL^{™} 301 medium containing 2% glutamine at a concentration between 5X10⁵ to 1.5X10⁶ cells/ml.

1.5 grams of LMP agarose were dissolved in 100 ml of assay medium (DMEM/F12 medium containing 2% glutamine) in a microwave oven. The agarose was placed in a 47°C water bath until use. The cells were collected from suspension culture and were re-suspended in 60 ml of assay medium for determination of quantity of cells. The cell suspension was then further diluted to a final concentration of 2.5x10⁷ cells/6 ml. The 6 ml of cell suspension and the melted agarose were mixed at 1:1 v/v and the mixture was then immediately poured into a white OMNI PLATE^{®} tray. In experiments where 3 cells lines were mixed together in a lawn, the concentration was 2.5×10⁷ cells/2 ml for each cell line resulting in a total of 6 ml of cell suspension when the lines were combined. The agarose lawn of cells was then allowed to solidify at room temperature.

Solutions of peptides were then delivered to the lawn using a HDR 96-pin tool that delivers approximately 10 nanoliters (nl) of solution. After the addition of peptide, the lawns are incubated at 37°C, 5% CO₂ in a tissue culture incubator for 4 hours. 10 ml of a 1:1 mixture of LUCLITE PLUS^{™} reagent and PBS containing 1 mM CaCl₂/ 1 mM MgCl₂ was added to each OMNI PLATE^{®} tray at room temperature under subdued light conditions. After 10 minutes, the excess liquid on top of the agarose lawn was removed. Luminescence was detected using a VIEWLUX^{®} camera.

Images were acquired by focusing luminescent light from the OMNI PLATE^{®} tray, for 90 seconds, onto a CCD camera chip via a telecentric lens. Digitized images were downloaded to a computer for image analysis using the ROBOIMAGE^{™} image analysis software package. Briefly, a 50 x 75 cell grid was superimposed on the image and the average gray intensity value grid unit is transferred into a Microsoft EXCEL^{®} spreadsheet. The gray intensity was determined by scanning each grid unit to determine where the highest point in the region was located. The surrounding 9 grid units were averaged and this represented the z value for a given luminescent signal. The x and y co-ordinates were recorded to facilitate the production of graphical representations of the data.

The response of CHO-6xCRE-luc⁺ cells expressing MC1, GLP-1 or CRH1 receptors to [NDP]-αMSH (1-13), GLP-1 (7-36), and CRH (1-41) is shown in Figures 5A-7B. Figures 5A, 6A, and 7A depict the image of luminescence emanating from the locations where solution-containing peptide was delivered to the surface of the lawn. Rows 1, 2 and 3 (top to bottom of plate) contain concentration curves for [NDP]-α-MSH, GLP-1, and CRH, respectively. Approximately 10 nanoliters (nl) of solutions containing, from left to right, 5x10⁻⁴ M, 1.5x10⁻⁴ M, 5x10⁻⁵, 1.5x10⁻⁵ M, 5x10⁻⁶ M, and 1.5x10⁻⁶ M peptide was dispensed to the surface of the agarose lawn using the HDR pin tool. A concentration-response curve of luminescence activity was only observed when peptide was applied to the cell line expressing the appropriate receptor (e.g. GLP-1 peptide to the cell line expressing GLP-1 receptor). Note that the area of luminescence decreased with increasing size of the peptide, which could be attributed to decreased diffusion of the larger peptides.

Figures 5B, 6B, and 7B are line graph representations of the concentrations curves for each peptide. The response values were calculated by subtracting the background z values for the lawn from the z values for the area where peptide was delivered. These data support the use of a lawn format of cell lines stably expressing both a GPCR and a reporter gene construct in the assay for GPCR agonists.

Figures 8A and 8B depict the data obtained by mixing together the three stable cell lines expressing the MC1, GLP-1 or CRH receptors. The concentration-response curve data obtained with the cell mixture was very similar to the data shown in Figures 5A-7B. Thus, stable cell lines expressing a reporter gene and GPCRs can be mixed together in a lawn format for the simultaneous assay of GPCR agonists.

### Example 8

### Bead-Based Assay

Agonists for GPCRs were assayed using beads to which peptides or compounds are attached by a photolinker. The beads were either mixed in the agarose or were applied to the surface of the lawn. The attached compound or peptide was cleaved by exposure to UV light.

Cell culture and assay methods were as described in Example 1. Hexamer peptides were linked to 200 micrometer TENTAGEL^{™} microsphere beads (polystyrene core with TENTAGEL^{™} pegs - available from Rapp Polymere GmbH of Tübingen, Germany) by a photocleavable linker. Two peptides were tested for validation purposes: Peptide 1 - Ac-NH-Ser-Val-dHis-dPhe-Arg-Trp (SEQ ID NO:1) and Peptide 2 - Ac-NH-Val-Ala-dHis-dPhe-Arg-Tyr (SEQ ID NO:2). Beads were added to the agarose lawn either by pushing the bead into the agarose with forceps or by suspending the beads in the melted agarose/cell mixture. Peptides were cleaved from the beads by exposure to ultraviolet light using a UV lamp set at 365 nm wavelength (λ).

A time course of the effect of ultraviolet light exposure (λ = 365 nm) on the response of a cell line expressing a reporter gene construct and the MC1 receptor to increasing concentrations of [NDP]-α-MSH is shown in Figure 9. The peptide concentrations and delivery were the same as described in Example 1. UV light exposure up to 32 minutes did not significantly affect the response to the peptide:

A time course of delivery of MC1R agonist peptides 1 and 2, which were linked to beads (described in methods), to a lawn of CHO-6CRE-luc⁺-MC1R cells is shown in Figures 10A-10E. The peptides were cleaved from the beads by increasing the time of UV exposure. Figures 10A-10E depict UV exposure for 0, 0.5, 1, 3, and 10 minutes, respectively. A concentration-response curve for of [NDP]-α-MSH was run in row 1 on each lawn at the same concentrations as in Figure 9. Five (5) beads each of peptide 1 and 2 were pushed into the agarose layer with forceps. Peptide 1 and 2 were on the left and right of each lawn, respectively.

Figure 11A is a line graph representation of the average luminescence of the 5 beads at each time point. The response was calculated by subtracting the background z value for the lawn from the z value for each area to which the bead was applied. The magnitude of response is dictated by both the response of the reporter cell line and the amount of peptide that can be released from the beads. Both peptide 1 and 2 are full agonists for MC1R when tested in a well-based reporter assay so both peptides are capable of eliciting a response similar to [NDP]-α-MSH.

Figure 11A is a line graph representation of the [NDP]-α-MSH concentration curve from Figure 10E. The maximal response value was approximately 25. The response to both peptides 1 and 2 began to plateau at the 3-minute time point (Figure 11B) when the response value was well below the maximal response to [NDP]-α-MSH (Figure 11A). Thus, the plateau in the magnitude of response is largely dependent on the amount of peptide that can be released from these particular beads. In the case of peptide 1, the maximal response could have also been dependent on the maximal response of the reporter gene system; however, this is certainly not the case for peptide 2. Figure 12 is a three-dimensional representation of the data from the 10-minute time point. The shaded bands from the top to bottom of each cone represent the weakening of signal as the peptide diffuses away from the initial point of delivery.

Figure 13A depicts an experiment in which peptide 1 and 2 beads were mixed with the agarose and cells before the lawn was poured. The hardened lawn containing the cells and beads was then exposed to UV light for 10 minutes. The areas of luminescence were the areas that contained beads. Figure 13B shows a contour graph of this data. Using this type of representation facilitates location of the center of the area of luminescence, and therefore, the location of the bead containing active peptide, which can be picked for retests and confirmation. These data support the assay of compounds or peptides that are delivered by release from beads. Furthermore, the beads can either be applied to the lawn or mixed together with the agarose and cells prior to pouring the lawn.

### Example 9

### Evaluation of White OMNI PLATE^{®} Trays

Opaque white OMNI PLATE^{®} trays specially designed for the lawn assay as described in methods were compared with commercially available clear OMNI PLATE^{®} trays. The white trays were produced by adding titanium dioxide to the virgin polystyrene resin. As shown in Figure 14, it was observed that the signal to noise ratio was vastly improved using the white trays versus the clear trays. Thus, the white trays were used in Examples 7 and 8 above.

### Example 10

### Evaluation of LUCLITE^{™} Reagent

The question of whether the LUCLITE^{™} reagent might cause some breakdown of the compounds or codes linked to beads was raised. To address this question, the following experiment was performed.

The beads that contain a phenyl acetic acid capped analytical construct comprising a code portion (unique ratio of isotopically labeled amino acids) and a compound portion were tested. The beads with constructs linked to them were incubated for 0 minutes, 10 minutes, 1 hour, 2 hours and overnight with diluted LUCLITE^{™} reagent (1:1 LUCLITE ^{™} reagent::PBS). The beads were then treated with DMF for 5 minutes. The construct was then released from the beads with trifluoroacetic acid and triethylsilane. By mass spectrometry analysis it was determined that that the LUCLITE^{™} reagent solution did not affect the integrity of either the code or compound portion of the construct.

This Example indicates that there is a long window of time between when the LUCLITE^{™} reagent is added and when the beads need to be picked out of the lawn after the trays have been imaged.

### REFERENCES

The references listed below as well as all references cited in the specification are incorporated herein by reference to the extent that they supplement, explain, provide a background for or teach methodology, techniques and/or compositions employed herein.

Bevan, N., Scott, S., Shaw, P.E., Lee, M.G., Marshall, F.H., Rees, S. (1998) Nociception activates Elk-1 and Sap1a following expression of the ORL1 receptor in Chinese hamster ovary cells. *Neuroreport* 9:2703-2708.

Goetz A.S., Andrews, J.L., Littleton, T.R., Ignar, D.M. (2000) Development of a facile method for high throughput screening with reporter gene assays. *J. Biomolec. Screening* 5:377-384.

Himmler, A., Stratowa, C., Czernilofsky, A.P. (1995) Use of a luciferase reporter system for characterizing G-protein linked receptors. *Curr. Opin. Biotechnol.* 6:574-581.

Ignar, D.M., Rees, S. (2000) The study of drug-receptor interactions using reporter gene systems in mammalian cells. In Kenakin, T., Angus, J.A. (eds.), *The Pharmacology of Functional, Biochemical, and Recombinant Receptor Systems, Handbook of Experimental Pharmacology.* Springer-Verlag, Heidelberg, Vol. 148:391-412.

Stratowa, C., Himmler, A., Czernilofsky, A.P. (1995a) Use of a luciferase reporter system for characterizing G-protein linked receptors. *Curr. Opin. Biotechnol.* 6:574-581.

Stratowa, C. Machat, H., Burger, E., Himmler, A., Schafer, R., Spevak, W., Weyer, U., Wiche-Casatanon, M., Czernilofsky, A.P. (1995b) Functional characterization of the human neurokinin receptors NK1, NK3, and NK3 based on cellular assay system. *J. Recept. Signal Transduct. Res.* 15:617-630.

Weyer, U., Schafer, R., Himmler, A., Mayer, S.K., Burger, E., Czernilofsky, A.P. Stratowa C. (1993) Establishment of a cellular assay system for G protein-linked receptors: Coupling of human NK2 and 5-HT₂ receptors to phospholipase C activates a luciferase reporter gene. *Receptors Channels* 1:193-200.

The application of which this description and claims form a part can be used as a basis for priority in respect of any subsequent application. The claims of such subsequent application can be directed to any feature or combination of features described herein. They can take the form of product, composition, process or use claims and can include, by way of example and without limitation, one or more of the following claims.

It will be understood that various details of the invention can be changed without departing from the scope of the invention. Furthermore, the foregoing description is for the purpose of illustration only, and not for the purpose of limitation-the invention being defined by the claims.

## Claims

1. A method for screening candidate compounds for an ability to modulate the biological activity of a target, the method comprising:
(a) providing a substrate comprising a cell that expresses the target and comprising an indicator, the substrate adapted for receiving at least about 384 candidate compounds;
(b) contacting the substrate with a plurality of liquid candidate compound samples using a PIN tool to form a predetermined pattern of candidate compound samples on the substrate, whereby the candidate compounds within the candidate compound samples interact with the target within the predetermined pattern;
(c) detecting a signal within the predetermined pattern, the signal produced by the indicator upon interaction between the target and a candidate compound; and
(d) identifying a candidate compound as a modulator of the biological activity of the target based upon an amount of signal produced as compared to a control sample.

2. The method of claim 1, wherein the substrate further comprises a matrix selected from the group consisting of a biological matrix medium, a synthetic matrix medium, and combinations thereof.

3. The method of claim 2, wherein the biological matrix medium further comprising a material selected from the group consisting of agarose, collagen, laminin, basement membrane-derived complex, derivatives thereof, and combinations thereof.

4. The method of claim 3, wherein the agarose is low-melt agarose.

5. The method according to any preceding claim, wherein the indicator is selected from the group consisting of a fluorogenic compound, a fluorescent compound, a chemiluminescent compound, a colorimetric compound, a radiolabeled compound and combinations thereof.

6. The method according to any preceding claim, wherein the cell expresses the target, the indicator, or both the target and the indicator.

7. The method of claim 6, wherein the cell expresses two or more targets.

8. The method according to any preceding claim, wherein the cell is selected from the group consisting of a *Xenopus* melanocyte, a mammalian cell, a bacterial cell, a fungal cell and combinations thereof.

9. The method according to any preceding claim, further comprising a combination of cells, wherein each cell within the combination of cells expresses one or more targets.

10. The method according to any preceding claim, further comprising:
(a) contacting the substrate with a ligand for the target after loading the candidate compounds, whereby the candidate compounds interact with the target in the presence of the ligand within the predetermined pattern;
(b) detecting a signal within the predetermined pattern, the signal produced by the indicator upon blocking of an interaction between the target and the ligand; and
(c) identifying a candidate compound as a modulator of the biological activity of the target based upon an amount of signal produced as compared to a control sample.

11. The method according to any preceding claim, further comprising a plurality of cells, wherein the cells are mixed throughout the substrate.

12. The method according to any one of claims 1 - 10, further comprising a plurality of cells, wherein the cells form a layer in the substrate.

13. The method of claim 12, wherein the substrate further comprises a matrix layer formed on the layer of cells in the substrate, wherein the matrix layer is positioned to receive the plurality of candidate compound samples.

14. The method according to any preceding claim, wherein the plurality of liquid candidate compound samples comprises a number equivalent to a multiplier of a number of liquid candidate compound samples available from a source.

15. The method of claim 14, wherein the multiplier is a number ranging from 1 to 25.

16. The method of claim 15, wherein the number of liquid candidate compound samples available from the source is 96, 384 or 1,536.

17. The method according to any preceding claim, wherein the signal is detected at a plurality of time points after contacting the substrate with the plurality of liquid candidate compound samples.

18. The method of claim 17, wherein the step of identifying a candidate compound as a modulator of the biological activity of the target comprises a subtraction of a signal produced by the candidate compound at an initial time point from a signal produced by the candidate compound at a final time point.

19. The method according to any preceding claim, wherein the amount of signal produced by a candidate compound is evaluated by analyzing a diameter of the signal, a volume of the signal, an area of the signal, a concavity of the signal, a circularity of the signal or combinations thereof.

20. The method according to any preceding claim, wherein the substrate is contacted with the plurality of liquid candidate compound samples whereby the liquid candidate compound samples touch the surface of the substrate and a tool used to place the liquid candidate compound samples proximate to the substrate does not contact the substrate.

21. The method according to any one of claims 1 to 19, wherein the substrate is contacted with the plurality of liquid candidate compound samples whereby the liquid candidate compound samples and a tool used to deliver the liquid candidate compound samples to the substrate each contact the substrate.

## Patentansprüche

1. Verfahren zur Durchmusterung von Kandidatenverbindungen auf eine Fähigkeit zur Modulierung der biologischen Aktivität eines Ziels, wobei das Verfahren die folgenden Schritte umfaßt:
(a) Bereitstellen eines Substrats, das eine Zelle umfaßt, die das Ziel exprimiert, und das einen Indikator umfaßt, wobei das Substrat zum Aufnehmen von wenigstens circa 384 Kandidatenverbindungen angepaßt ist;
(b) Inkontaktbringen des Substrats mit einer Mehrzahl von flüssigen Kandidatenverbindungsproben unter Verwendung eines PIN-Werkzeugs zur Bildung eines vorgegebenen Musters von Kandidatenverbindungsproben auf dem Substrat, wodurch die Kandidatenverbindungen innerhalb der Kandidatenverbindungsproben mit dem Ziel innerhalb des vorgegebenen Musters wechselwirken;
(c) Detektieren eines Signals innerhalb des vorgegebenen Musters, wobei das Signal durch den Indikator bei Wechselwirkung zwischen dem Ziel und einer Kandidatenverbindung erzeugt wird; und
(d) Identifizieren einer Kandidatenverbindung als Modulator der biologischen Aktivität des Ziels auf Basis einer Signalstärke, die im Vergleich mit einer Kontrollprobe erzeugt wird.

2. Verfahren gemäß Anspruch 1, worin das Substrat ferner eine Matrix umfaßt, die aus der Gruppe ausgewählt ist, die aus einem biologischen Matrixmedium, einem synthetischen Matrixmedium und Kombinationen daraus besteht.

3. Verfahren gemäß Anspruch 2, worin das biologische Matrixmedium ferner ein Material umfaßt, das aus der Gruppe ausgewählt ist, die aus Agarose, Collagen, Laminin, aus Basalmembran stammendem Komplex, Derivaten davon und Kombinationen daraus besteht.

4. Verfahren gemäß Anspruch 3, worin die Agarose niedrigschmelzende Agarose ist.

5. Verfahren gemäß jedem vorhergehenden Anspruch, worin der Indikator aus der Gruppe ausgewählt ist, die aus einer fluorogenen Verbindung, einer fluoreszenten Verbindung, einer chemilumineszenten Verbindung, einer kolorimetrischen Verbindung, einer radiomarkierten Verbindung und Kombinationen daraus besteht.

6. Verfahren gemäß jedem vorhergehenden Anspruch, worin die Zelle das Ziel, den Indikator oder sowohl das Ziel als auch den Indikator exprimiert.

7. Verfahren gemäß Anspruch 6, worin die Zelle zwei oder mehr Ziele exprimiert.

8. Verfahren gemäß jedem vorhergehenden Anspruch, worin die Zelle aus der Gruppe ausgewählt ist, die aus einem Xenopus-Melanozyt, einer Säugerzelle, einer bakteriellen Zelle, einer Pilzzelle und Kombinationen daraus besteht.

9. Verfahren gemäß jedem vorhergehenden Anspruch, das ferner eine Kombination von Zellen umfaßt, worin jede Zelle innerhalb der Kombination von Zellen ein oder mehrere Ziele exprimiert.

10. Verfahren gemäß jedem vorhergehenden Anspruch, das ferner die folgenden Schritte umfaßt:
(a) Inkontaktbringen des Substrats mit einem Liganden für das Ziel nach dem Aufladen der Kandidatenverbindungen, wodurch die Kandidatenverbindungen mit dem Ziel in Gegenwart des Liganden innerhalb des vorgegebenen Musters wechselwirken;
(b) Detektieren eines Signals innerhalb des vorgegebenen Musters, wobei das Signal durch den Indikator bei Blockierung einer Wechselwirkung zwischen dem Ziel und dem Liganden erzeugt wird; und
(c) Identifizieren einer Kandidatenverbindung als Modulator der biologischen Aktivität des Ziels auf Basis einer Signalstärke, die im Vergleich mit einer Kontrollprobe erzeugt wird.

11. Verfahren gemäß jedem vorhergehenden Anspruch, das ferner eine Mehrzahl von Zellen umfaßt, worin die Zellen im Substrat vermischt sind.

12. Verfahren gemäß einem der Ansprüche 1 bis 10, das ferner eine Mehrzahl von Zellen umfaßt, worin die Zellen eine Schicht im Substrat bilden.

13. Verfahren gemäß Anspruch 12, worin das Substrat ferner eine Matrixschicht umfaßt, die auf der Schicht von Zellen im Substrat gebildet ist, worin die Matrixschicht angeordnet ist, um die Mehrzahl von Kandidatenverbindungsproben aufzunehmen.

14. Verfahren gemäß jedem vorhergehenden Anspruch, worin die Mehrzahl von flüssigen Kandidatenverbindungsproben eine Anzahl umfaßt, die äquivalent zu einem Multiplikator einer Anzahl von flüssigen Kandidatenverbindungsproben ist, die aus einer Quelle erhältlich sind.

15. Verfahren gemäß Anspruch 14, worin der Multiplikator eine Zahl im Bereich von 1 bis 25 ist.

16. Verfahren gemäß Anspruch 15, worin die Anzahl von flüssigen Kandidatenverbindungsproben, die aus der Quelle erhältlich sind, 96, 384 oder 1.536 ist.

17. Verfahren gemäß jedem vorhergehenden Anspruch, worin das Signal zu einer Mehrzahl von Zeitpunkten nach Inkontaktbringen des Substrats mit der Mehrzahl von flüssigen Kandidatenverbindungsproben detektiert wird.

18. Verfahren gemäß Anspruch 17, worin der Schritt des Identifizierens einer Kandidatenverbindung als Modulator der biologischen Aktivität des Ziels eine Subtraktion eines Signals, das durch die Kandidatenverbindung zu einem Anfangszeitpunkt erzeugt wird, von einem Signal umfaßt, das durch die Kandidatenverbindung zu einem Endzeitpunkt erzeugt wird.

19. Verfahren gemäß jedem vorhergehenden Anspruch, worin die durch eine Kandidatenverbindung erzeugte Signalstärke durch Analysieren eines Durchmessers des Signals, eines Volumens des Signals, einer Fläche des Signals, einer Wölbung des Signals, einer Rundheit des Signals oder Kombinationen daraus ausgewertet wird.

20. Verfahren gemäß jedem vorhergehenden Anspruch, worin das Substrat mit der Mehrzahl von flüssigen Kandidatenverbindungsproben in Kontakt gebracht wird, wodurch die flüssigen Kandidatenverbindungsproben die Oberfläche des Substrats berühren und ein Werkzeug, das zum Plazieren der flüssigen Kandidatenverbindungsproben in die Nähe des Substrats verwendet wird, nicht das Substrat berührt.

21. Verfahren gemäß einem der Ansprüche 1 bis 19, worin das Substrat mit der Mehrzahl von flüssigen Kandidatenverbindungsproben in Kontakt gebracht wird, wodurch die flüssigen Kandidatenverbindungsproben und ein Werkzeug, das zur Übertragung der flüssigen Kandidatenverbindungsproben auf das Substrat verwendet wird, jeweils das Substrat berühren.

## Revendications

1. Procédé pour sélectionner des composés candidats pour une aptitude à moduler l'activité biologique d'une cible, procédé comprenant les étapes consistant à :
(a) fournir un substrat comprenant une cellule qui exprime la cible et comprenant un indicateur, le substrat étant adapté à recevoir au moins environ 384 composés candidats ;
(b) mettre en contact le substrat avec une pluralité d'échantillons de composés candidats liquides en utilisant un instrument PIN pour former un motif prédéterminé d'échantillons de composés candidats sur le substrat, ce qui fait que les composés candidats dans les échantillons de composés candidats interagissent avec la cible dans le motif prédéterminé ;
(c) détecter un signal dans le motif prédéterminé, le signal étant produit par l'indicateur par interaction entre la cible et un composé candidat ; et
(d) identifier un composé candidat comme modulateur de l'activité biologique de la cible sur la base d'une quantité de signal produite par comparaison avec un échantillon témoin.

2. Procédé suivant la revendication 1, dans lequel le substrat comprend en outre une matrice choisie dans le groupe consistant en un milieu de matrice biologique, un milieu de matrice synthétique et leurs associations.

3. Procédé suivant la revendication 2, dans lequel le milieu de matrice biologique comprend en outre une matière choisie dans le groupe consistant en l'agarose, le collagène, la laminine, un complexe dérivé de la membrane basale, leurs dérivés et leurs associations.

4. Procédé suivant la revendication 3, dans lequel l'agarose est un agarose à bas point de fusion.

5. Procédé suivant l'une quelconque des revendications précédentes, dans lequel l'indicateur est choisi dans le groupe consistant en un composé fluorogène, un composé fluorescent, un composé chimioluminescent, un composé colorimétrique, un composé radiomarqué et leurs associations.

6. Procédé suivant l'une quelconque des revendications précédentes, dans lequel la cellule exprime la cible, l'indicateur ou bien à la fois la cible et l'indicateur.

7. Procédé suivant la revendication 6, dans lequel la cellule exprime deux ou plus de deux cibles.

8. Procédé suivant l'une quelconque des revendications précédentes, dans lequel la cellule est choisie dans le groupe consistant en un mélanocyte de *Xenopus,* une cellule de mammifère, une cellule bactérienne, une cellule fongique et leurs associations.

9. Procédé suivant l'une quelconque des revendications précédentes, comprenant en outre une association de cellules, chaque cellule dans l'association de cellules exprimant une ou plusieurs cibles.

10. Procédé suivant l'une quelconque des revendications précédentes, comprenant en outre les étapes consistant à :
(a) mettre en contact le substrat avec un ligand pour la cible après avoir chargé les composés candidats, les composés candidats interagissant ainsi avec la cible en présence du ligand dans le motif prédéterminé ;
(b) détecter un signal dans le motif prédéterminé, le signal étant produit par l'indicateur lors du blocage d'une interaction entre la cible et le ligand ; et
(c) identifier un composé candidat comme modulateur de l'activité biologique de la cible sur la base d'une quantité de signal produite par comparaison avec un échantillon témoin.

11. Procédé suivant l'une quelconque des revendications précédentes, comprenant en outre une pluralité de cellules, les cellules étant mélangées dans la totalité du substrat.

12. Procédé suivant l'une quelconque des revendications 1 à 10, comprenant en outre une pluralité de cellules, les cellules formant une couche dans le substrat.

13. Procédé suivant la revendication 12, dans lequel le substrat comprend en outre une couche de matrice formée sur la couche de cellules, dans le substrat, la couche de matrice étant positionnée de manière à recevoir la pluralité d'échantillons de composés candidats.

14. Procédé suivant l'une quelconque des revendications précédentes, dans lequel la pluralité d'échantillons de composés candidats liquides comprend un nombre équivalent à un multiplicateur d'un nombre d'échantillons de composés candidats liquides disponibles à partir d'une source.

15. Procédé suivant la revendication 14, dans lequel le multiplicateur est un nombre allant de 1 à 25.

16. Procédé suivant la revendication 15, dans lequel le nombre d'échantillons de composés candidats liquides disponibles à partir de la source est de 96, 384 ou 1536.

17. Procédé suivant l'une quelconque des revendications précédentes, dans lequel le signal est détecté à une pluralité de points de temps après la mise en contact du substrat avec la pluralité d'échantillons de composés candidats liquides.

18. Procédé suivant la revendication 17, dans lequel l'étape d'identification d'un composé candidat comme modulateur de l'activité biologique de la cible comprend une soustraction d'un signal produit par le composé candidat à un point de temps initial d'un signal produit par le composé candidat à un point de temps final.

19. Procédé suivant l'une quelconque des revendications précédentes, dans lequel la quantité de signal produite par un composé candidat est évaluée en analysant un diamètre du signal, un volume du signal, une surface du signal, une concavité du signal, une circularité du signal ou leurs associations.

20. Procédé suivant l'une quelconque des revendications précédentes, dans lequel le substrat est mis en contact avec la pluralité d'échantillons de composés candidats liquides, ce qui fait que les échantillons de composés candidats liquides touchent la surface du substrat et qu'un outil utilisé pour placer les échantillons de composés candidats liquides à proximité du substrat n'entre pas en contact avec le substrat.

21. Procédé suivant l'une quelconque des revendications 1 à 19, dans lequel le substrat est mis en contact avec la pluralité d'échantillons de composés candidats liquides, ce qui fait que les échantillons de composés candidats liquides et un outil utilisé pour délivrer les échantillons de composés candidats liquides au substrat entrent chacun en contact avec le substrat.
